# EUROPEAN PATENT APPLICATION

(11) **EP 3 744 734 A1**
(43) Date of publication of application: **02.12.2020**
(21) Application number: 19740714.1
(22) Date of filing: 21.01.2019
(51) Int. Cl.: C07K 16/28, C12N 15/13, A61K 39/395, A61P 35/00

(54) **ANTI-4-1BB ANTIBODY, ANTIGEN-BINDING FRAGMENT THEREOF AND MEDICAL USE THEREOF**

(30) Priority: 22.01.2018 CN 201810058076; 04.12.2018 CN 201811472564
(71) Applicant: Jiangsu Hengrui Medicine Co., Ltd., Lianyungang, Jiangsu 222047 (CN)
(72) Inventor: HUANG, Hao, Lianyungang, Jiangsu 222047 (CN); JIANG, Jiahua, Lianyungang, Jiangsu 222047 (CN); YAN, Shude, Lianyungang, Jiangsu 222047 (CN); CAO, Guoqing, Lianyungang, Jiangsu 222047 (CN); ZHANG, Lianshan, Lianyungang, Jiangsu 222047 (CN)
(74) Representative: Sonzogni, Laura Gabriella
(86) International application number: PCT/CN2019/072484
(87) International publication number: WO 2019/141268

(57) **Abstract**

Provided are an anti-4-1BB antibody, an antigen-binding fragment thereof and medical use thereof. Further, provided are a chimeric antibody and a humanized antibody comprising a CDR region of the anti-4-1BB antibody, as well as a pharmaceutical composition comprising a human anti-4-1BB antibody or an antigen-binding fragments thereof, and use thereof as anti-cancer medicaments. In particular, provided is use of a humanized anti-4-1BB antibody in the preparation of a medicament for the treatment of diseases or conditions mediated by 4-1BB.

## Description

### FIELD OF THE INVENTION

The present application relates to an anti-4-1BB antibody and antigen-binding fragments, a chimeric antibody, a humanized antibody comprising the CDR regions of the anti-4-1BB antibody, and a pharmaceutical composition comprising the human anti-4-1BB antibody or antigen-binding fragment thereof, and its use as an anticancer agent.

### BACKGROUND OF THE INVENTION

Cancer is a huge health challenge for human society for a long time.

Traditional therapies (such as surgery, chemotherapy, and radiotherapy) have shown little effect on treating disseminated solid tumors. Tumor immunotherapy is a hot spot in the field of cancer therapy, in which T cell immunotherapy is in a central position. Tumor immunotherapy fully utilizes and recruits killer T cells in tumor patients to kill tumors. Tumor immunotherapy may be the most effective and safest way for the treatment of tumors. Tumor immunotherapy currently has good prospects for the treatment of several different types of cancer, including disseminated metastatic tumors.

Two signaling pathways are involved in activation of T cells in human body. In addition to a first signal provided by presenting MHC-antigen peptides to T cells via antigen presenting cells (APCs), a second signal, provided by a series of costimulatory molecules, is also necessary for T cells to produce a normal immune response. This dual signaling pathway system plays a vital role in the balance of the immune system *in vivo.* It strictly regulates the immune responses to autoantigens and non-autoantigens. In the absence of a second signal provided by costimulatory molecules, non-response or sustained specific immune response of T cells will result in tolerance.

4-1BB (also known as CD137, TNFRSF9) is a transmembrane protein of the tumor necrosis factor receptor superfamily (TNFRS). Human 4-1BB has a protein of 255 amino acids, including a signal sequence (amino acid residues 1-17), an extracellular domain (169 amino acids), a transmembrane domain (27 amino acids), and an intracellular domain (42 amino acid). 4-1BB is expressed as a monomer or a dimer on the cell surface, and it mediates signal transduction via trimerization upon binding to its ligand (4-1BBL).

4-1BB is usually activation-dependent and is widely present in immune cells, including activated NK and NKT cells, regulatory T cells, dendritic cells (DC), stimulated mast cells, differentiated myeloid cells, monocytes, neutrophils, and eosinophils. Studies have shown that initiation of 4-1BB can enhance cell proliferation, survival, and cytokine production.

Agonistic antibodies against 4-1BB increase the expression of co-stimulatory molecules in many models and significantly enhance the response of cytolytic T lymphocytes, resulting in anti-tumor efficacy; whereas durable anti-tumor protective T cell memory responses have also been observed in tumor models with monotherapy and combination therapy targeting 4-1BB.

For the still unmet medical needs associated with 4-1BB, several international pharmaceutical companies are actively developing monoclonal antibodies against 4-1BB. Related patents such as WO2000029445, WO2005035584, WO2012032433, WO2003049755, WO2017205745A and the like. To date, anti-4-1BB antibodies provided by Bristol-Myers Squibb (BMS) and Pfizer's have entered Phase I/II clinical trials, and the similar products from Pieris have also entered Phase I clinical trials.

The present application is directed to providing anti-4-1BB antibodies with high affinity, high selectivity and high biological activity, and medicaments, compositions and methods thereof, for use in cancer therapy by stimulating 4-1BB and its pathway to lead to immune activation.

### SUMMARY OF THE INVENTION

The application provides an anti-4-1BB antibody or antigen-binding fragment thereof, comprising:
an antibody light chain variable region comprising at least one LCDR selected from the group consisting of SEQ ID NO: 6, SEQ ID NO: 7, SEQ ID NO: 8; SEQ ID NO: 14, SEQ ID NO: 15 and SEQ ID NO: 16; and
an antibody heavy chain variable region comprising at least one HCDR selected from the group consisting of SEQ ID NO: 3, SEQ ID NO: 4, SEQ ID NO: 5, SEQ ID NO :11, SEQ ID NO: 12, SEQ ID NO: 13 and SEQ ID NO: 45.

In a specific embodiment of the above anti-4-1BB antibody or antigen-binding fragment thereof according to present application, wherein the antibody light chain variable region comprisesLCDR1, LCDR2 and LCDR3 as set forth in SEQ ID NO: 6, SEQ ID NO: 7 and SEQ ID NO: 8, respectively.

In a specific embodiment of the above anti-4-1BB antibody or antigen-binding fragment thereof according to present application, wherein the antibody light chain variable region comprisesLCDR1, LCDR2 and LCDR3 as set forth in SEQ ID NO: 14, SEQ ID NO: 15 and SEQ ID NO: 16, respectively.

In a specific embodiment of the above anti-4-1BB antibody or antigen-binding fragment thereof according to present application, wherein the antibody heavy chain variable region comprises HCDR1, HCDR2 and HCDR3 as set forth in SEQ ID NO: 3, SEQ ID NO: 45 and SEQ ID NO: 5, respectively, or comprises HCDR1, HCDR2 and HCDR3 as set forth in SEQ ID NO: 3, SEQ ID NO: 4 and SEQ ID NO: 5, respectively.

In a specific embodiment of the above anti-4-1BB antibody or antigen-binding fragment thereof according to present application, wherein the antibody heavy chain variable region comprises HCDR1, HCDR2 and HCDR3 as set forth in SEQ ID NO: 11, SEQ ID NO: 12 and SEQ ID NO: 13, respectively

In a specific embodiment of the above anti-4-1BB antibody or antigen-binding fragment thereof according to present application, wherein the antibody light chain variable region comprises:
LCDR1, LCDR2, and LCDR3 as set forth in SEQ ID NO: 6, SEQ ID NO: 7 and SEQ ID NO: 8, respectively; or
LCDR1, LCDR2, and LCDR3 as set forth in SEQ ID NO: 14, SEQ ID NO: 15 and SEQ ID NO: 16, respectively; and
the antibody heavy chain variable region comprises:
   HCDR1, HCDR2 and HCDR3 as set forth in SEQ ID NO: 3, SEQ ID NO: 45 and SEQ ID NO: 5, respectively; or
   HCDR1, HCDR2 and HCDR3 as set forth in SEQ ID NO: 3, SEQ ID NO: 4 and SEQ ID NO: 5, respectively; or
   HCDR1, HCDR2 and HCDR3 as set forth in SEQ ID NO: 11, SEQ ID NO: 12 and SEQ ID NO: 13, respectively.

In a specific embodiment of the present application, the anti-4-1BB antibody or antigen-binding fragment thereof may be selected from any one of the following:
(1) the antibody light chain variable region comprises LCDR1, LCDR2 and LCDR3 as set forth in SEQ ID NO: 6, SEQ ID NO: 7, and SEQ ID NO: 8, respectively; and the antibody heavy chain variable region comprises HCDR1, HCDR2 and HCDR3 as set forth in SEQ ID NO: 3, SEQ ID NO: 45 and SEQ ID NO: 5, respectively; or
(2) the antibody light chain variable region comprises LCDR1, LCDR2 and LCDR3 as set forth in SEQ ID NO: 14, SEQ ID NO: 15, and SEQ ID NO: 16, respectively; and the antibody heavy chain variable region comprises HCDR1, HCDR2 and HCDR3 as set forth in SEQ ID NO: 11, SEQ ID NO: 12 and SEQ ID NO: 13, respectively.

In a specific embodiment of the present application, the antibody light chain variable region sequence is set forth in SEQ ID NO: 2; and the heavy chain variable region sequence is set forth in SEQ ID NO: 1.

In a specific embodiment of the present application, the antibody light chain variable region sequence is set forth in SEQ ID NO: 10; and the heavy chain variable region sequence is set forth in SEQ ID NO: 9.

The application also provides an anti-4-1BB antibody or antigen-binding fragment thereof, comprising:
antibody light chain variable region comprising LCDR1, LCDR2 and LCDR3 as set forth in SEQ ID NO: 42, SEQ ID NO: 43 and SEQ ID NO: 44, respectively; and
antibody heavy chain variable region comprising HCDR1, HCDR2 and HCDR3 as set forth in SEQ ID NO: 39, SEQ ID NO: 40 and SEQ ID NO: 41, respectively.

The present application also provides a 4-1BB antibody or antigen-binding fragment, wherein the light chain variable region LCVR of the antibody or antigen fragment is set forth in SEQ ID NO: 38, and the heavy chain variable region HCVR is set forth in SEQ ID NO. :37.

In a specific embodiment of the present application, the anti-4-1BB antibody or antigen-binding fragment thereof as described above is a murine antibody or fragment thereof.

In a specific embodiment of the present application, the antibody light chain variable region of the murine antibody or fragment thereof as described above, further comprises light chain FR regions of murine κ, λ chain or a variant thereof.

In a specific embodiment of the present application, the murine antibody or fragment thereof as described above, further comprises a light chain constant region of murine κ, λ chain or a variant thereof.

In a specific embodiment of the present application, the heavy chain variable region of the murine antibody or fragment thereof as described above further comprises heavy chain FR regions of murine IgG1, IgG2, IgG3, IgG4 or a variant thereof.

In a specific embodiment of the present application, the murine antibody or fragment thereof as described above, further comprises a heavy chain constant region of murine IgGl, IgG2, IgG3, IgG4 or a variant thereof.

In a specific embodiment of the present application, the anti-4-1BB antibody or antigen-binding fragment thereof as described above is a chimeric antibody or fragment thereof.

In a specific embodiment of the present application, the anti-4-1BB chimeric antibody or fragment thereof as described above, further comprises a light chain constant region of human kappa, lambda chain or a variant thereof.

In a specific embodiment of the present application, the anti-4-1BB chimeric antibody or fragment thereof as described above, further comprises a heavy chain constant region of human IgG1, IgG2, IgG3 or IgG4 or a variant thereof.

In a specific embodiment of the present application, the anti-4-1BB antibody or antigen-binding fragment thereof as described above is a human antibody or fragment thereof.

In a specific embodiment of the present application, the anti-4-1BB antibody or antigen-binding fragment thereof as described above is a humanized antibody or fragment thereof.

In a specific embodiment of the anti-4-1BB human antibody or fragment thereof as described above, wherein the light chain of the humanized antibody is set forth in SEQ ID NO: 18 or a variant thereof; the variant comprises from 0 to 10 amino acid changes in the light chain. The heavy chain sequence is set forth in SEQ ID NO: 17 or a variant thereof; the variant comprises from 0 to 10 amino acid changes in the heavy chain.

In a specific embodiment of the 4-1BB human antibody or fragment thereof as described above, wherein the light chain of the humanized antibody is set forth in SEQ ID NO: 20 or a variant thereof; the variant comprises from 0 to 10 amino acid changes in the light chain. The heavy chain sequence is set forth in SEQ ID NO: 19 or a variant thereof; the variant comprises from 0 to 10 amino acid changes in the heavy chain.

In a specific embodiment of the present application, the anti-4-1BB human antibody or fragment thereof as described above, further comprises a constant region of human IgG1, IgG2, IgG3 or IgG4 or a variant thereof, preferably comprises human IgG1 or IgG2 constant region.

In a specific embodiment of the present application, the anti-4-1BB antibody or antigen-binding fragment thereof as described above is a humanized antibody or a fragment thereof.

In a specific embodiment of the anti-4-1BB humanized antibody or fragment thereof as described above, the humanized antibody light chain variable region further comprises light chain FR regions of human κ, λ chain or a variant thereof.

In a specific embodiment of the above anti-4-1BB humanized antibody or fragment thereof, the light chain FR region sequence of the humanized antibody light chain variable region is derived from human germline light chain IGkV3-11 sequence as set forth in SEQ ID NO: 22; or derived from human germline light chain IGkV4-1 sequence as set forth in SEQ ID NO: 24.

In a specific embodiment of the above anti-4-1BB humanized antibody or fragment thereof, wherein the humanized antibody light chain variable region sequence is set forth in SEQ ID NO: 30 or SEQ ID NO: 36, or a variant thereof.

In a specific embodiment of the above anti-4-1BB humanized antibody or fragment thereof, the humanized antibody light chain sequence is set forth in SEQ ID NO: 18 or SEQ ID NO: 20, or a variant thereof.

In a specific embodiment of the above anti-4-1BB humanized antibody or fragment thereof, the variant of the humanized antibody light chain variable region preferably has from 0 to 10 (1, 2, 3, 4, 5, 6, 7, 8, 9, 10) amino acid changes in the light chain variable region; more preferably on amino acid positions 4, 47 and 62 or any combination thereof; more preferably M4L, P47A, V62I mutation and any combination thereof; more preferably P47A and V62I mutations, M4L and V62I mutations.

In a specific embodiment of the present application, the anti-4-1BB humanized antibody or fragment thereof as described above, further comprises a light chain constant region of a human kappa, lambda chain or a variant thereof.

In a specific embodiment of the present application, the humanized antibody heavy chain variable region of the above anti-4-1BB humanized antibody or fragment thereof further comprises heavy chain FR regions of human IgG1, IgG2, IgG3, IgG4 or a variant thereof.

In a specific embodiment of the present application, the heavy chain FR region of the heavy chain variable region of the above anti-4-1BB humanized antibody or fragment thereof is derived from human germline heavy chain IGHV3-30 sequence as set forth in SEQ ID NO: 21; or derived from human germline heavy chain IGHV 1-46 sequence as set forth in SEQ ID NO: 23.

In a specific embodiment of the present application, the humanized antibody heavy chain variable region of the above anti-4-1BB humanized antibody or fragment thereof is set forth in SEQ ID NO: 27 or SEQ ID NO: 33, or a variant thereof.

In a specific embodiment of the present application, the humanized antibody heavy chain of the above anti-4-1BB humanized antibody or fragment thereof is set forth in SEQ ID NO: 17 or SEQ ID NO:19, or a variant thereof; the variant preferably has from 0 to 10 amino acid changes in the heavy chain variable region; more preferably has mutation at amino acid position 48, 53, 75, 95 or 112 or any combination thereof; more preferably has mutation of M48I, E53D, A75S, Y95F, Q112T/R112Q or any combination thereof; more preferably E53D, A75S and R112Q; more preferably M48I, Y95F and Q112T.

In a specific embodiment of the present application, the above anti-4-1BB humanized antibody or fragment thereof, further comprises a heavy chain constant region of human IgG1, IgG2, IgG3 or IgG4 or a variant thereof, preferably, comprises heavy chain FR regions of human IgGl, IgG2 or IgG4, more preferably comprises heavy chain FR regions of human IgGl or IgG2.

In a specific embodiment of the present application, provided an anti-4-1BB humanized antibody or fragment thereof, comprising a light chain and a heavy chain:
the heavy chain comprising a heavy chain variable region selected from the group consisting of SEQ ID No. 25, 26, 27, 31, 32 and 33; and
the light chain comprising a light chain variable region selected from the group consisting of SEQ ID No. 28, 29, 30, 34, 35 and 36.

In a specific embodiment of the present application, the light chain variable region of the anti-4-1BB humanized antibody or fragment thereof as described above is set forth in SEQ ID NO: 36, and the heavy chain variable region thereof is set forth in SEQ ID NO:33.

In a specific embodiment of the present application, the light chain variable region of the anti-4-1BB humanized antibody or fragment thereof as described above is set forth in SEQ ID NO: 30, and the heavy chain variable region thereof is set forth in SEQ ID NO:27.

In a specific embodiment of the present application, the light chain of the anti-4-1BB humanized antibody or fragment thereof as described above is set forth in SEQ ID NO: 20, and the heavy chain thereof is set forth in SEQ ID NO: 19.

In a specific embodiment of the present application, the light chain of the anti-4-1BB humanized antibody or fragment thereof as described above is set forth in SEQ ID NO: 18, and the heavy chain thereof is set forth in SEQ ID NO: 17.

In a specific embodiment of the above anti-4-1BB antibody or antigen-binding fragment thereof, wherein the antigen-binding fragment of is Fab, Fv, sFv, F(ab')₂, linear antibody, single chain antibody, scFv, sdAb, sdFv, Nanobody, peptibody, domain antibody and multispecific antibody (bispecific antibody, diabody, triabody and tetrabody, tandem di-scFv, tandem tri-scFv).

The application further provides an isolated monoclonal antibody or antigen-binding fragment thereof that competes for binding to 4-1BB or epitope thereof with the monoclonal antibody or antigen-binding fragment thereof described above.

The application further provides a polynucleotide encoding the anti-4-1BB antibody or antigen-binding fragment thereof as described above, the polynucleotide may be DNA or RNA.

The application further provides an expression vector comprising the polynucleotide as described above, the expression vector may be a viral vector, and the virus may be an oncolytic virus.

The application further provides a host cell transformed with the expression vector as described above.

In a specific embodiment of the present application, the host cell as described above, characterized in that the host cell is a bacterium, preferably *Escherichia coli.*

In a specific embodiment of the present application, the host cell as described above is yeast, preferably *Pichia pastoris.*

In a specific embodiment of the present application, the host cell as described above is mammalian cell, preferably Chinese hamster ovary (CHO) cell or human embryonic kidney (HEK) 293 cell.

The present application also provides a multispecific antibody comprising a light chain variable region and a heavy chain variable region as described above.

The application also provides a single chain antibody comprising a light chain variable region and a heavy chain variable region as described above.

The application also provides an antibody-drug conjugate, comprising a light chain variable region and a heavy chain variable region as described above. The antibody-drug conjugate is formed by linking the antibody-linker-drug (toxin). Well-known linkers include cleavable linker, non-cleavable linker, for example, including but not limited to, SMCC, SPDP, and the like; The toxins are also well known in the art, such as DM1, DM4, MMAE, MMAF, and the like.

The present application also provides a method for producing the anti-4-1BB antibody or antigen-binding fragment thereof, comprising the steps of expressing the antibody or antigen-binding fragment thereof in the host cell as described above, and isolating the antibody or antigen-binding fragment thereof from the host cell.

The application further provides a pharmaceutical composition comprising theanti-4-1BB antibody or antigen-binding fragment thereof as described above and a pharmaceutically acceptable excipient, diluent or carrier.

The present application further provides use of any one or combination of the following in the preparation of a medicament: the anti-4-1BB antibody or antigen-binding fragment thereof according to the present application, the pharmaceutical composition according to the present application and the antibody-drug conjugate according to the present application; wherein the medicament is for treating or preventing a 4-1BB or 4-1BBL mediated disease or condition; the disease is preferably cancer; and the cancer is most preferably selected from the group consisting of melanoma, breast cancer, ovarian cancer, prostate cancer, pancreatic cancer, kidney cancer, lung cancer, liver cancer, stomach cancer, colorectal cancer, bladder cancer, head and neck cancer, thyroid cancer, esophageal cancer, cervical cancer, sarcoma, multiple myeloma, leukemia, lymphoma, gallbladder cancer and glioblastoma.

The present application further provides a method of treating or preventing a 4-1BB or 4-1BBL mediated disease or condition, comprising administering to a subject a therapeutically or prophylactically effective amount of the anti-4-1BB antibody or antigen-binding fragment thereof according to the present application, or the pharmaceutical composition according to the present application, or the antibody-drug conjugate according to the present application; wherein the disease is preferably cancer; and the cancer is most preferably selected from the group consisting of melanoma, breast cancer, ovarian cancer, prostate cancer, pancreatic cancer, kidney cancer, lung cancer, liver cancer, stomach cancer, colorectal cancer, bladder cancer, head and neck cancer, thyroid cancer, esophageal cancer, cervical cancer, sarcoma, multiple myeloma, leukemia, lymphoma, gallbladder cancer and glioblastoma.

### DESCRIPTION OF THE DRAWINGS

Figure 1: *In vitro* capture ELISA assay of the murine antibodies, indicating that all the anti-4-1BB murine antibodies to be tested from each hybridomas efficiently bind to human 4-1BB-his.
Figure 2: ELISA assay of the murine antibodies in blocking 4-1BBL ligand. The results showed that B1E7 and B1A7 antibodies were ligand-blocking types, while B1E10 and the two reference antibodies were non-blocking types.
Figure 3: 4-1BB reporter gene activation assay of the murine antibodies, indicating that the activation intensity was ranked in the order of B1E10>MOR7480 reference>B1E7>B1A7.
Figure 4: PBMC cell proliferation function activation assay of the two humanized antibodies. The results showed that the activation intensity of huB1E7 and huB1E10 was higher than that of reference MOR7480, wherein huB1E7 was relatively stronger.
Figure 5: 4-1BB reporter gene activation assay of the humanized antibodies. The results showed that the activation intensity was ranked in an order consistent with that for murine antibodies, which is B1E10>MOR7480 reference>B1E7.
Figure 6: Tumor growth curve in hu4-1BB transgenic C57BL/6 mice transplanted with MC38 intestinal cancer cells after administration of each anti-4-1BB antibody to be tested.
Figure 7: Body weight change curve of hu4-1BB transgenic C57BL/6 mice transplanted with MC38 intestinal cancer cells after administration of each anti-4-1BB antibody to be tested.

### DETAILED DESCRIPTION OF THE DISCLOSURE

### 1. Terminology

In order to more readily understood the invention, certain technical and scientific terms are specifically defined below. Unless specifically defined elsewhere in this document, all other technical and scientific terms used herein have the meaning commonly understood by one of ordinary skill in the art to which this invention pertains.

As used herein, the single-letter code and the three-letter code for amino acids are as described in J. Biol. Chem, 243, (1968) p3558.

As used herein, the term "antibody" refers to immunoglobulin, which is a four-peptide chain structure formed by linking two identical heavy chains and two identical light chains by disulfide bonds. Different immunoglobulin heavy chain constant regions exhibit different amino acid compositions and sequence orders, thereby presenting different kinds of antigenicity. Accordingly, immunoglobulins can be divided into five categories, or called immunoglobulin isotypes, namely IgM, IgD, IgG, IgA and IgE, their heavy chains are µ chain, δ chain, γ chain, α chain and ε chain, respectively. According to its amino acid composition of hinge region and the number and location of heavy chain disulfide bonds, the same type of Ig can be divided into different sub-categories, for example, IgG can be divided into IgG1, IgG2, IgG3, and IgG4. Light chain can be divided into κ or λ chain considering of different constant regions. Each of the five Igs can have κ or λ chain.

In the present application, the antibody light chain variable region mentioned herein further comprises a light chain constant region, which comprises a human or murine κ, λ chain or a variant thereof.

In the present invention, the antibody heavy chain variable region mentioned herein further comprises a heavy chain constant region, which comprises human or murine IgG1, 2, 3, 4 or a variant thereof.

Near the N-terminal of the antibody heavy chains and light chains sequence, about 110 amino acid sequence varies largely, known as the variable region (V region); the rest of the amino acid sequence near the C-terminus is relative stable, known as the constant region (C region).Variable region comprises three hypervariable regions (HVR) and four framework region (FR)having relatively conserved sequence. The three hypervariable regions determine the specificity of the antibody, also known as a complementarity determining region (CDR). Each light chain variable region (LCVR) and each heavy chain variable region (HCVR) is composed of three CDRs and four FRs, with order from the amino terminal to the carboxyl terminal being: FR1, CDR1, FR2, CDR2, FR3, CDR3, and FR4. Three light chain CDRs refer to LCDR1, LCDR2, and LCDR3; three heavy chain CDRs refer to HCDR1, HCDR2 and HCDR3. The numbers and locations of CDR amino acid residues in LCVR and HCVR of the antibody or the antigen-binding fragment herein correspond with known Kabat numbering criteria (LCDR1-3, HCDR2-3), or correspond with kabat and chothia (ABM) numbering criteria (HCDR1).

The term "recombinant human antibody" includes human antibodies prepared, expressed, created or isolated by recombinant methods, and the techniques and methods involved are well known in the art, such as:
(1) an antibody isolated from a human immunoglobulin gene transgenic or transchromosomal animal (e.g., a mouse), or a hybridoma prepared therefrom;
(2) an antibody isolated from transformed host cells expressing the antibody, such as a transfectoma;
(3) an antibody isolated from a recombinant combinatorial human antibody library; and
(4) an antibody prepared, expressed, created or isolated by splicing human immunoglobulin gene sequences to another DNA sequences or the like.

Such recombinant human antibody comprises a variable region and a constant region, such regions involve specific human germline immunoglobulin sequences encoded by germline genes, but also involve subsequent rearrangements and mutations such as those occur during the antibody maturation.

The term "murine antibody" in the present application refers to monoclonal antibody against human-4-1BB or epitope thereof, which is prepared according to the knowledge and skills in the art. During the preparation, a test object is injected with 4-1BB antigen, and then hybridoma expressing antibody which possesses desired sequence or functional characteristics is separated. In a particular embodiment of the present application, the murine 4-1BB antibody or the antigen-binding fragment thereof further comprises a light chain constant region of murine κ, λ chain or a variant thereof, or further comprises a heavy chain constant region of murine IgG1, IgG2, IgG3 or IgG4, or a variant thereof.

The term "human antibody" includes antibodies having variable and constant regions with human germline immunoglobulin sequences. Human antibodies of the present application may include amino acid residues that are not encoded by human germline immunoglobulin sequences (e.g., mutations introduced by random or site-specific mutagenesis in vitro or by somatic mutation in vivo). However, the term "human antibody" does not include an antibody in which CDR sequences derived from other mammalian species germline(such as mouse germline) have been grafted onto a human framework sequence (i.e., "humanized antibody")..

The term "humanized antibody", also known as CDR-grafted antibody, refers to an antibody generated by grafting murine CDR sequences into a variable region framework of a human antibody. Humanized antibody overcomes the strong antibody response induced by the chimeric antibody which carries a large amount of murine protein components. To avoid the decrease of activity along with the decrease of immunogenicity, the variable region of the human antibody is subjected to a minimum back mutation to maintain the activity.

The term "chimeric antibody", is an antibody which is formed by fusing the variable region of a murine antibody with the constant region of a human antibody, the chimeric antibody can alleviate the murine antibody-induced immune response. To establish a chimeric antibody, hybridoma secreting specific murine monoclonal antibody is firstly established, a variable region gene is cloned from mouse hybridoma cells, then a constant region gene of a human antibody is cloned as desired, the mouse variable region gene is ligated with the human constant region gene to form a chimeric gene which can be inserted into a human vector, and finally the chimeric antibody molecule is expressed in the eukaryotic or prokaryotic industrial system. The constant region of a human antibody is selected from the heavy chain constant region of human IgG1, IgG2, IgG3 or IgG4 or a variant thereof, preferably the heavy chain constant region of human IgG2 or IgG4, or that of IgG1 which has no ADCC (antibody-dependent cell-mediated cytotoxicity) after amino acid mutation.

As used herein, "antigen-binding fragment" refers to a Fab fragment, a Fab' fragment, a F(ab')2 fragment with antigen-binding activity, as well as a Fv fragment sFv fragment binding to human 4-1BB. Fv fragment is a minimum antibody fragment carrying all antigen-binding sites, it comprises a heavy chain variable region, a light chain variable region, but without constant region. Generally, Fv antibody further comprises a polypeptide linker between the VH and VL domains, and is capable of forming a structure necessary for antigen binding. Also, different linkers can be used to connect the variable regions of two antibodies to form a polypeptide, named as single chain antibody or single chain Fv (sFv).

As used herein, "binding to 4-1BB", refers to the ability to interact with 4-1BB or an epitope thereof, which may be of human origin. The term "antigen binding site" as used herein refers to discontinuous three-dimensional sites on the antigen, recognized by the antibody or the antigen-binding fragment of the present application.

The term "epitope" refers to the sites on an antigen that specifically bind to an immunoglobulin or antibody. The epitope can be formed by adjacent amino acids, or by non-adjacent amino acids which have been brought to be closer due totertiary folding of a protein. The Epitope formed by adjacent amino acids is typically retained after exposure to denaturing solvents, whereas the epitope formed by tertiary folding is typically lost after treatment with denaturing solvents. Epitopes typically include at least 3-15 amino acids in a unique spatial conformation. Methods for determining epitope bound by a given antibody are well known in the art, including immunoblotting and immuno precipitation assays, and the like. Methods for determining the spatial conformation of an epitope include techniques in the art and techniques described herein, such as X-ray crystallography and two-dimensional nuclear magnetic resonance.

The term "specifically binds to", "selectively binds to" as used herein, refers to the binding of an antibody to an epitope on a predetermined antigen. Typically, the antibody binds to a predetermined antigen or the epitope thereof at approximately less than 10⁻⁷ M or even less equilibrium dissociation constant (K_{D}), and the affinity of the antibody for binding to the predetermined antigen or epitope thereof is at least two times higher than that for non-specific antigens (such as BSA) other than the predetermined antigen (or epitope thereof) or than that for closely related antigens, as measured in an instrument via surface plasmon resonance (SPR) techniques, wherein the recombinant human 4-1BB or its epitope is used as an analyte and the antibody is used as a ligand. The term "an antibody recognizing the antigen" can be used interchangeably herein with the term "specifically binding antibody".

The term "cross reaction" refers to the ability of the antibody of the present application to bind to 4-1BB from a different species. For example, an antibody of the present application that binds to human 4-1BB can also bind to 4-1BB from another species. Cross-reactivity is measured by detecting the specific reactivity with purified antigens in binding assays (e.g., SPR and ELISA), or by detecting the binding or functional interaction with cells physiologically expressing 4-1BB. Methods for determining cross-reactivity include standard binding assays as described herein, such as surface plasmon resonance analysis, or flow cytometry.

The terms "inhibition" or "blockade" are used interchangeably and encompass both partial and complete inhibition/ blockade. Inhibition/ blockade of 4-1BB preferably reduces or alters the normal level or type of activity that is generated upon binding to 4-1BB without inhibition or blockade. Inhibition and blockade are also intended to include a measurable decrease of binding affinity for 4-1BB when contacted with an anti-4-1BB antibody, compared to the binding affinity for 4-1BB in absence of anti-4-1BB antibody.

The term "inhibiting the growth" (e.g., involving cells) is intended to include any measurable reduction in cell growth.

The terms "inducing an immune response" and "enhancing an immune response" are used interchangeably and refer to the immune response to the stimulation of a particular antigen(i.e., passive or adaptive). The term "inducing", with respect to CDC or ADCC, refers to stimulating a specific mechanism to directly kill cells.

As used herein, the term "ADCC", namely antibody-dependent cell-mediated cytotoxicity, refers to cells expressing Fc receptors directly kill target cells coated by an antibody through recognizing the Fc segment of the antibody. ADCC effector function of the antibody can be reduced or eliminated via modification of the Fc segment in IgG. The modification refers to mutations of the antibody heavy chain constant region, such as mutations selected from N297A, L234A, L235A in IgG1; IgG2/4 chimera; F235E, or L234A/E235A mutations in IgG4.

Methods for producing and purifying antibodies and antigen-binding fragments are well known in the art and can be found, for example, in Antibodies, A Laboratory Manual, Cold Spring Harbor, Chapter 5-8 and 15. For example, mice can be immunized with human 4-1BB, or fragments thereof, and the resulting antibodies can then be renatured, purified and sequenced using conventional methods well known in the art. Antigen-binding fragments can also be prepared by conventional methods. The antibody or the antigen-binding fragment of the present invention is genetically engineered to add one or more human framework regions (FRs) to non-human derived CDRs. Human FR germline sequences can be obtained from ImMunoGeneTics(IMGT) via their website http://imgt.cines.fr, or from The Immunoglobulin FactsBook, 2001ISBN012441351.

The engineered antibody or antigen-binding fragment of the present invention may be prepared and purified using conventional methods. For example, cDNA sequences encoding a heavy chain (SEQ ID NO: 17) and a light chain (SEQ ID NO: 18) may be cloned and recombined into a GS expression vector. The recombined immunoglobulin expression vector may then stably transfect CHO cells. As a more recommended method well known in the art, mammalian expression system may result in glycosylation of antibodies, typically at the highly conserved N-terminus in the F_{C} region. Stable clones may be obtained through expression of an antibody specifically binding to human antigen. Positive clones may be expanded in a serum-free culture medium for antibody production in bioreactors. Culture medium, into which an antibody has been secreted, may be collected and purified by conventional techniques. The antibody may be subjected to filtration and concentration using common techniques. Soluble mixtures and multimers may be effectively removed by common techniques, including molecular sieve or ion exchange. The obtained product may be immediately frozen, for example at -70°C, or may be lyophilized.

The antibody of the present invention is a monoclonal antibody. Monoclonal antibody or mAb, as used herein, refers to an antibody that is derived from a single clone including but not limited to any eukaryotic, prokaryotic, or phage clone. Monoclonal antibodies and antigen-binding fragments thereof can be recombined, for example, by hybridoma technologies, recombinant technologies, phage display technologies, synthetic technologies (e.g., CDR-grafting), or other technologies known in the art.

"administration" and "treatment," as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "administration" and "treatment" can refer, e.g., to therapeutic, pharmacokinetic, diagnostic, research, and experimental methods. Treatment of a cell encompasses contacting a reagent with the cell, as well as contacting a reagent with a fluid, where the fluid is in contact with the cell. "administration" and "treatment" also mean in vitro and ex vivo treatments, e.g., of a cell, by a reagent, diagnostic, binding composition, or by another cell. "Treatment," as it applies to a human, veterinary, or a research subject, refers to therapeutic treatment, prophylactic or preventative measures, to research and diagnostic applications.

"Treat" means to the administration of a therapeutic agent (such as a composition comprising any of the antibodies of antigen-binding fragments thereof of the present invention) internally or externally to a subject suffering from, suspected to suffer from or having tendency to suffer from one or more disease symptoms for which the agent has known therapeutic activity. Typically, the therapeutic agent is administered in an amount effective to alleviate one or more disease symptoms in the treated subject or population, either by inducing the regression of or inhibiting the progression of such symptom(s) to any clinically measurable degree. The amount of a therapeutic agent that is effective to alleviate any particular disease symptom (also referred to "therapeutically effective amount") may vary according to factors such as the disease state, age, and weight of the subject, and the ability of the drug to elicit a desired response in the subject. Whether a disease symptom has been alleviated can be assessed by any clinical measurement typically used by physicians or other skilled healthcare providers to assess the severity or progression status of that symptom. While an embodiment of the present application (e.g., a treatment method or article of manufacture) may not be effective in alleviating the disease symptom(s) of interest in every subject, it should alleviate the target disease symptom(s) of interest in a statistically significant number of subjects as determined by any statistical test known in the art such as the Student's t-test, the chi-square test, the U-test according to Mann and Whitney, the Kruskal-Wallis test (H-test), Jonckheere-Terpstra-test and the Wilcoxon-test.

"Effective amount" encompasses an amount sufficiently to ameliorate or prevent a symptom or sign of a medical condition. Effective amount also means an amount sufficiently to allow or facilitate diagnosis. An effective amount for a particular subject or veterinary subject may vary depending on factors such as the condition being treated, the general health of the subject, the route and dose of administration and the severity of side effects. An effective amount can be the maximal dose or dosing protocol that avoids significant side effects or toxic effects.

"Exogenous" refers to substances that are produced outside an organism, cell, or human body, depending on the context. "Endogenous" refers to substances that are produced within a cell, organism, or human body, depending on the context.

"Identity" refers to sequence similarity between two polynucleotide sequences or between two polypeptides. When a position in the two sequences to be compared is occupied by the same base or amino acid monomer subunit, e.g., if a position in each of two DNA molecules is occupied by adenine, then the molecules are homologous at that position. The percent of identity between two sequences is a function of the number of matched or homologous positions shared by the two sequences divided by the number of positions to be compared,×100. For example, if 6 of 10 positions in two sequences are matched or homologous when the sequences are optimally aligned, then the two sequences share60% identity. Generally, the comparison is made when two sequences are aligned to give maximum percent identity.

As used herein, the expressions "cell," "cell line," and "cell culture" are used interchangeably and all such designations include progeny thereof. Thus, the words "transformants" and "transformed cells" include the primary subject cell and cultures derived therefrom without considering the number of passages. It is also understood that all progeny may not be precisely identical in DNA content, due to deliberate or random mutations. Mutant progeny obtained by screening, which have the same function or biological activity as that of originally transformed cell, are also contemplated. Where distinct designations are intended, it will be clear from the context.

"Optional" or "optionally" means that the event or situation that follows may but does not necessarily occur, and the description includes the instances in which the event or circumstance does or does not occur. For example, "optionally comprises 1-3 antibody heavy chain variable regions" means that the antibody heavy chain variable region with specific sequence can be, but not necessarily, be present.

"Pharmaceutical composition" refers to one containing a mixture of one or more antibodies or antigen-binding fragments thereof according to the present invention or a physiologically/pharmaceutically acceptable salt or prodrug thereof with other chemical components, as well as additional components such as physiologically/pharmaceutically acceptable carriers and excipients. The pharmaceutical composition aims at promoting the administration to an organism, facilitating the absorption of the active ingredient and thereby exerting a biological effect.

Hereinafter, the present application is further described with reference to examples; however, the scope of the present application is not limited thereto. In the examples of the present invention, where specific conditions are not described, the experiments are generally conducted under conventional conditions as described in Antibodies, A Laboratory Manual and Molecular Cloning Manual, by Cold Spring Harbor, or under conditions proposed by the material or product manufacturers. Where the source of the reagents is not specifically given, the reagents are commercially available conventional reagents.

### Example 1: Preparation of antibodies

The anti-human 4-1BB monoclonal antibody library was produced by immunizing mice. Eight-week-old BalB/C and A/J strain female mice (Comparative Medical Center of Yangzhou University, animal production license number: SCXK (Su) 2017-007) were used in the experiments. Feeding environment: SPF level. After the mice were purchased, the animals were kept in the laboratory for 1 week, with 12/12-hour light/dark cycle, at temperature of 20-25°C, and with a humidity of 40-60%.

The antigen for immunization was human 4-1BB recombinant protein with Fc tag (human 4-1BB-Fc), purchased from Acro Biosystems, Cat No #41B-H5258: expressed in HEK293, AA Gln 25-Gln 186 (http://www.acrobiosystems.cn/P150-Human_4-1BB_%7CTNFRSF9_ProteinC-Fc_Tag HEK293_expressed.html; Accession number #NP_001552.2). The antigen was emulsified with Freund's adjuvant (Sigma,Cat No: F5881/F5506): the first immunization was performed with Freund's complete adjuvant, and the rest booster immunizations were performed with Freund's incomplete adjuvant. The ratio of antigen to adjuvant was 1:1, and 25 µg protein/200 µl/mouse was injected for each immunization. The emulsified antigen was inoculated via subcutaneous injections at multiple sites (generally 6-8 sites on the back) and an injection at a single site on the toe palm, on day 0, 21, 35, and 49 (first immunization and 3 booster immunizations). The last booster immunization was performed 3 days before the splenocyte fusion, via intraperitoneal (IP) injection of 50 µg protein/mice of the antigen solution formulated with physiological saline.

On day 42 and 56, the blood samples were collected from the immunized mice and the antisera were isolated. The sera of the mice were subjected to ELISA method described in Example 2, to determine the mouse serum titer of the antibody and to determine the neutralizing activity in blocking 4-1BB/4-1BBLbinding.

After the fourth immunization, the mice with higher serum titer of the antibody and having higher activity in blocking 4-1BB/4-1BBL were selected for splenocyte fusion. Spleen lymphocytes were fused with myeloma cell Sp2/0 cells (ATCC® CRL-8287™) using optimized PEG-mediated fusion procedure, and then the fused cells were plated into 96-well plates and cultured in HAT medium for 10 to 14 days until the growth of hybridoma cells were observed. About 2 weeks after the cell fusion, the binding reactivity of the hybridoma culture medium to 4-1BB recombinant protein was detected by indirect ELISA assay described in Example 2, and the positive hybridoma cell supernatant was able to recognize and bind to 4-1BB recombinant protein.

The 4-1BB positive hybridoma cells identified by the primary screening were picked out, transferred to a new 96-well plate, and numbered according to the position of the fusion plate. After further culture for 3-4 days, the culture supernatants were collected and were subjected to indirect ELISA assay described in Test Example 1 to confirm the positive wells again. Once the culture medium was collected, the medium was replenished. For the positive wells, the supernatants were collected again for cell-based functional screening 3-4 days later, to select hybridoma cells secreting activated anti-4-1BB antibody. Brief procedures for functional screening were as follows: collecting cell strains with A 4-1BB reporter (HEK293F-h4-1BB-NFkb) which were in logarithmic growth phase and seeding into 96-well plates at volume of 20 µl/well (comprising approximately 10,000 cells). Adding mouse Fc-specific secondary antibody (final concentration of 15 nM, for cross-linking the supernatant antibody) and 60 µlcell supernatant or antibody solution to be tested, incubating for 5 hours, measuring the activity of luciferase with ONE-Glo™ Luciferase Assay System (Promega, E6120), and selecting the primary hybridoma cells in which the anti-4-1BB antibody was activated according to the measured values. In the experiment, the antibody Urelumab (BMS) and MOR7480 (Pfizer) were used as positive references, and hIgG was used as a negative reference. The screening data of the top 17 hybridoma were shown in the table below.

**Table 1. Hybridoma screening data**

| No. | Hybridoma No | luciferase activity |
|---|---|---|
| 1 | B1E10 | 2519.06 |
| 2 | B1E7 | 1097.93 |
| 3 | B1A7 | 964.85 |
| 4 | B1H9 | 803.25 |
| 5 | B1C8 | 779.48 |
| 6 | 3C1 | 769.14 |
| 7 | B1H1 | 760.47 |
| 8 | 3F5 | 674.77 |
| 9 | 5G1 | 673.10 |
| 10 | B1B9 | 670.17 |
| 11 | B1H2 | 665.41 |
| 12 | 8D5 | 660.61 |
| 13 | B1G1 | 655.91 |
| 14 | B1C9 | 632.14 |
| 15 | 6A1 | 626.03 |
| 16 | B1G12 | 622.64 |
| 17 | 2H11 | 607.20 |
| PC | Urelumab (10nM) | 2193.01 |
| PC | MOR 7480 (10nM) | 1001.57 |
| NC | hIgG (10nM) | 289.55 |

The three most preferably original hybridoma cells were limiting diluted by two rounds of subcloning to obtain the monoclonal cell lines (B1E10, B1E7 and B1A7), which were then cloned and sequenced. Total cellular RNAs were obtained by conventional RNA extraction techniques, and then the PCR products of the monoclonal antibody variable regions were obtained by reverse transcription polymerase chain reaction (RT-PCR). The PCR products were separated and recovered on agarose gel, and then were cloned into a gene vector and transformed into Escherichia coli. Several transformed colonies were randomly selected and were subjected to PCR amplification to amplify the monoclonal antibody variable region for gene sequencing.

The heavy and light chain variable region sequences of murine mAb B1E10 were as follows:
B1E10 HCVR 
EVQLVESGGGLVEPGGSLKLSCAASGFTFGDYYMYWVRQTPEKRLEWVATISD
GGSYTYYPDNVKGRFTISRDNAKNSLDLQMSHLKSEDTAMYYCARYYSKALYA
MDYWGRGTSVTVSS
SEQID NO: 1
B1E10 LCVR

DIVLTQSPASLAVSLGQRATLSCRASKSVSTSGFSYIHWYQQKPGQPPKLLIYTAS
NLESGVPARFSGSGSGTDFTLNIHPVEEEDAATYYCQHSRELPLTFGAGTKLEVK
SEQID NO: 2

It comprises the following CDR sequences:

**Table 2. CDR sequences**

| Name | sequence | SEQID NO |
|---|---|---|
| HCDR1 | GFTFGDYYMY | SEQID NO: 3 |
| HCDR2 | TISDGGSYTYYPDNVKG | SEQID NO: 4 |
| HCDR3 | YYSKALYAMDY | SEQID NO: 5 |
| LCDR1 | RASKSVSTSGFSYIH | SEQID NO: 6 |
| LCDR2 | TASNLES | SEQID NO: 7 |
| LCDR3 | QHSRELPLT | SEQID NO: 8 |

The heavy and light chain variable region sequences of murine mAb B1E7 were as follows:
B1E7 HCVR 
EVQLQQSGAELVRAGSSVEMSCKASGYTFTSYGLNWVKQRPGQGLEWIGYINP
GSGYTKYNEKFEGKTTLTVDKSSSTVYMQLRSLTSEDSAVYFCARWGLGRNWN
FAVWGTGTTVTVSS SEQID NO: 9
B1E7 LCVR

DIVLTQSPASLAVSLGQRATMSCRASESVDSYGNTFMHWYQQKPGQPPKLLIYR
ASNLESGIPARFSGSGSGTDFTLTINPVEADDVATYYCQQSNEDPLTFGSGTKLEI
K SEQID NO: 10

It comprises the following CDR sequences:

**Table 3. CDR sequences**

| Name | sequence | SEQID NO |
|---|---|---|
| HCDR1 | GYTFTSYGLN | SEQID NO: 11 |
| HCDR2 | YINPGSGYTKYNEKFEG | SEQID NO: 12 |
| HCDR3 | WGLGRNWNFAV | SEQID NO: 13 |
| LCDR1 | RASESVDSYGNTFMH | SEQID NO: 14 |
| LCDR2 | RASNLES | SEQID NO: 15 |
| LCDR3 | QQSNEDPLT | SEQID NO: 16 |

The heavy and light chain variable region sequences of murine mAb B1A7 were as follows:
B1A7 HCVR 
QVTLKESGPGILQPSQTLSLTCSFSGFSLSTSGMGVGWIRQPSGKGLEWLAHIW
WDDVKRYNPALKSRLTISKDTSSSQVFLKIASVDTADTATYYCAQMTSSVFAYW
GQGTLVTVSA SEQID NO: 37
B1A7 LCVR

DIVLTQSPDSLAVSLGQRATISCRASESVDSYGHSFMHWYQQKPGQPPKLLIYRA
SNLESGIPARFSGSGSRTDFTLTINVVEADDVATYYCHQSYEEPWTFGGGTKLEIK
SEQID NO: 38

It comprises the following CDR sequences:

**Table 4. CDR sequences**

| Name | sequence | SEQID NO |
|---|---|---|
| HCDR1 | GFSLSTSGMGVG | SEQID NO: 39 |
| HCDR2 | HIWWDDVKRYNPALKS | SEQID NO: 40 |
| HCDR3 | MTSSVFAY | SEQID NO: 41 |
| LCDR1 | RASESVDSYGHSFMH | SEQID NO: 42 |
| LCDR2 | RASNLES | SEQID NO: 43 |
| LCDR3 | HQSYEEPWT | SEQID NO: 44 |

### Example 2: Identification and screening of antibodies by ELISA assay

### 1. Binding assay via in vitro indirect ELISA:

Human 4-1BB-Fc protein was diluted to a concentration of 2 µg/ml with carbonate buffer pH 9.6, and added to a 96-well microtiter plate at a volume of 100 µl/well, and placed in an incubator at 37 °C for 2 hours. The plate was washed once with PBST (PBS containing 0.05% Tween-20, pH 7.4), added with 5% skim milk (skim milk powder, Bright Dairy) blocking solution diluted with PBST, 200 µl/well, and incubated at 37°C for 2 hours or at 4°C overnight (16-18 hours) for blocking. Once the blocking was finished, the blocking solution was discarded and the plate was washed 4 times with PBST buffer.

The test mouse sera (or antibodies, cell culture supernatants)were diluted to various concentrations with sample dilution solution containing 5% NHS (2.5% skim milk in PBST) (i.e., the final concentration of normal human IgG is 1 mg/ml), incubated at room temperature for 40 minutes, added into the microtiter plate, 100 µl/well, and incubated at 37 °C for 40 minutes. After the incubation, the plate was washed 4 times with PBST, added with 100 µl/well of HRP-labeled goat anti-mouse secondary antibody (Jackson Immuno Research, cat #115-036-071) diluted with PBST, and incubated at 37 °C for 40 minutes. The plate was washed 4 times with PBST, added with 100 µl/well of TMB chromogenic substrate (Huzhou Innoreagents Biotechnology Co., Ltd.), incubated at room temperature for 10-15 min in the dark, and added with 50 µl/well 1M H₂SO₄ to terminate the reaction.The absorbance was read at 450 nm under a microplate reader (Beijing Prang New Technology Co., Ltd., Model DNM-9602) and the resulting data were analyzed.

### 2. Binding assay via in vitro capture ELISA:

The goat anti-mouse IgG secondary antibody (Jackson Immuno Research, cat #115-006-071) was diluted to a concentration of 2 µg/ml with PBS buffer, pH 7.4, added into a 96-well microtiter plate, 100 µl/well, and placed in an incubator at 37 °C for 2 hours. The plate was washed once with PBST, added with 200 µl/well of 5% skim milk (skim milk powder, Bright Dairy) blocking solution diluted with PBST, and incubated at 37 °C for 2 hours or at 4 °C overnight (16-18 hours) for blocking. After the blocking was finished, the blocking solution was discarded and the plate was washed 4 times with PBST.

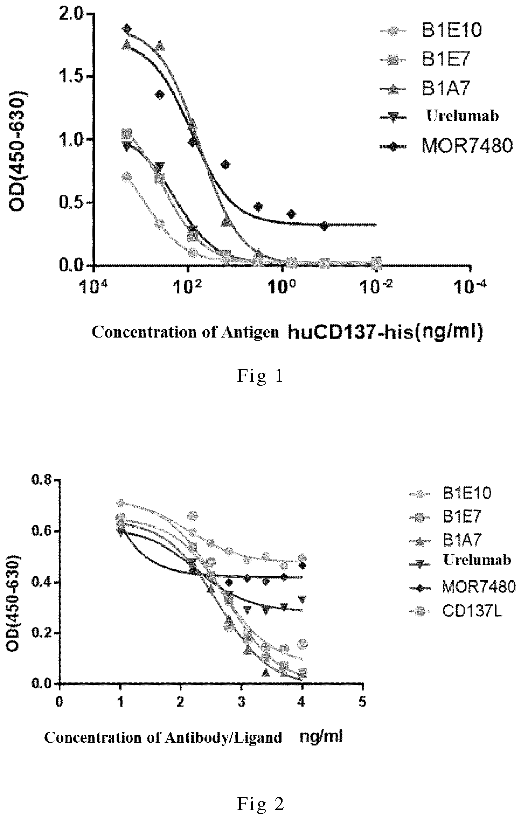

The test mouse sera or antibodies were diluted to various concentrations with 5% NHS sample dilution solution (2.5% skim milk in PBST), incubated at room temperature for 40 minutes, added into the plate, 100 µl/well, and incubated at 37°C for 40 minutes. After the incubation, the plate was washed 4 times with PBST, added with 100 µl/well of the biotinylated 4-1BB-his protein (Sinobiological, Beijing #10041-H08H) diluted with the sample dilution solution, and incubated at 37 °C for 40 minutes. After the incubation, the plate was washed 4 times with PBST, added with 100 µl/well of HRP-labeled streptavidin (Jackson Immuno Research, cat#016-030-084) diluted with PBST, and incubated at 37 °C for 40 minutes. The plate was washed 4 times with PBST, added with 100 µl/well of TMB chromogenic substrate (Huzhou Innoreagents Biotechnology Co., Ltd.), incubated at room temperature for 10-15 min in the dark, and added with 50 µl/well 1M H₂SO₄ to terminate the reaction. The absorbance was read at 450 nm under a microplate reader (Beijing Prang New Technology Co., Ltd., Model DNM-9602) and the resulting data were analyzed. The capture ELISA results of three hybridoma mouse antibodies and the Pfizer reference antibody MOR7480 and the BMS reference antibody Urelumab were shown in Figure 1. The EC₅₀ values were shown in the table below.

**Table 5. EC₅₀ values**

| Antibody | B1E10 | B1E7 | B1A7 | Urelumab | MOR7480 |
|---|---|---|---|---|---|
| EC₅₀ (ng/ml) | 880.9 | 332 | 55.95 | 191.9 | 83.97 |

### 3. Blocking the binding of 4-1BB to 4-1BBL by the antibodies via ELISA assay:

The 4-1BB ligand (i.e., Tumor necrosis factor ligand superfamily member 9, TNFSF9) is capable of binding to 4-1BB on the T cell surface and can activate T cell immune function as a positive costimulatory signal. In this assay, the selected anti-human 4-1BB antibodies were tested for their ability of blocking the binding of human 4-1BB protein to human 4-1BB ligand via an *in vitro* blocking assay.

Specifically, the human 4-1BB-his protein was coated onto a 96-well plate, added with anti-human 4-1BB antibodies (or mouse sera) and incubated, and added with human 4-1BBL-Fc protein, incubated and washed. The amount of 4-1BBL-Fc binding to 4-1BBcoated on the bottom of the plate was determined by HRP-labeled goat anti-human IgG secondary antibody (Jackson Immuno Research, cat#109-036-098), and the IC₅₀ values of the 4-1BB antibodies in blocking the 4-1BB active epitope were calculated.

The 4-1BB-his protein was diluted to a concentration of 2 µg/ml with a carbonate buffer pH 9.6, and added into a 96-well microtiter plate at a volume of 100 µl/well, and placed in an incubator at 37 °C for 2 hours. The plate was washed once with PBST, added with 200 µl/well of5% skim milk (skim milk powder, Bright Dairy) blocking solution diluted with PBST, and incubated at 37 °C for 2 hours or at 4 °C overnight (16-18 hours) for blocking. After the blocking was finished, the blocking solution was discarded and the plate was washed 4 times with PBST.

The test mouse sera or antibodies and internal control 4-1BBL were diluted to various concentrations with a sample diluent solution (2.5% skim milk in PBST), added into a plate, 100 µl/well, and incubated in an incubator at 37 °C for 40 minutes. After the incubation, the plate was washed 4 times with PBST, added with 100 µl/well of0.8 ng/ml 4-1BBL-Fc (Acro, #41L-H5257) protein diluted with a sample dilution solution, and incubated at 37 °C for 40 minutes. After the incubation, the plate was washed 4 times with PBST, added with 100 µl/well of HRP-labeled goat anti-human IgG secondary antibody (Jackson Immuno Research, cat #109-036-098) diluted with PBST, and incubated at 37 °C for 40 minutes. The plate was washed 4 times with PBST, added with 100 µl/well TMB chromogenic substrate (Huzhou Innoreagents Biotechnology Co., Ltd.), incubated at room temperature for 10-15 min in the dark, and added with 50 µl/well of 1M H₂SO₄ to terminate the reaction. The absorbance was read at 450 nm under a microplate reader (Beijing Prang New Technology Co., Ltd., Model DNM-9602) and the resulting data were analyzed. The results of the three hybridoma mouse antibodies and Pfizer reference antibody in blocking 4-1BBL were shown in Figure 2, and the EC₅₀ values were shown in the table below.

**Table 6. EC₅₀ values**

| | B1E10 | B1E7 | B1A7 | Urelumab | MOR7480 | 4-1BBL |
|---|---|---|---|---|---|---|
| EC₅₀ (ng/ml) | N/A | 591.9 | 416.9 | N/A | N/A | 162.1 |

As can be seen from the table, both B1E7 and B1A7 effectively block the epitope of 4-1BBL, while B1E10, MOR7480 and Urelumab cannot block the binding to 4-1BBL.

### Example 3: Construction and expression of anti-4-1BB recombinant chimeric antibodies

Site-directed amino acid mutations were introduced into FR region (framework region) of the heavy chain variable region (VH) and the light chain variable region (VL) of the murine antibody B1E10 and B1E7 of the present application, respectively. According to different combinations of amino acid mutations, different humanized antibody heavy and light chains were designed, and plasmids with different light and heavy chain combinations were transfected into cells to produce humanized antibodies.

The heavy chain vector was designed as follows: signal peptide + mutated heavy chain variable region sequence + human IgGl constant region sequence. The light chain vector was designed as follows: signal peptide + mutated light chain variable region sequence + human Kappa constant region sequence. The above sequences were inserted into pCEP4 vector, respectively. After the vector plasmids were confirmed, the plasmids were extracted and subjected to sequencing for verification. The validated plasmids were transfected into human 293F cells with PEI. The continuously cultured293F cells were cultured in serum-free medium (Shanghai Aupmaxi, OPM-293CD03) until reaching logarithmic growth phase, and used for cell transfection. 21.4 µg of the plasmid comprising the humanized antibody light chain and 23.6 µg of plasmid comprising the humanized antibody heavy chain were dissolved in 10 ml of Opti-MEM® I Reduced Serum Medium (GIBCO, 31985-070), mixed thoroughly, and then added with 200 µg of PEI, mixed, incubated at RT for 15 min, and added into 50mL of cells. The cell culture conditions: 5% CO2, 37 °C, 125 rpm/min. During the culture, supplementary material was added on day 1 and day 3, until the cell viability was less than 70%, and the cell supernatants were collected and centrifuged. The centrifuged cell culture medium was loaded onto an antibody purification affinity column, and washed with phosphate buffer, eluted with glycine-hydrochloric acid buffer (pH 2.7, 0.1M Gly-HCl), neutralized with 1 M Tris hydrochloric acid,pH9.0, and dialyzed against phosphate buffer to finally obtain the purified chimeric antibody ChB1E10 and ChB1E7.

### Example 4:In vitro cell reporter assay of anti-4-1BB antibodies

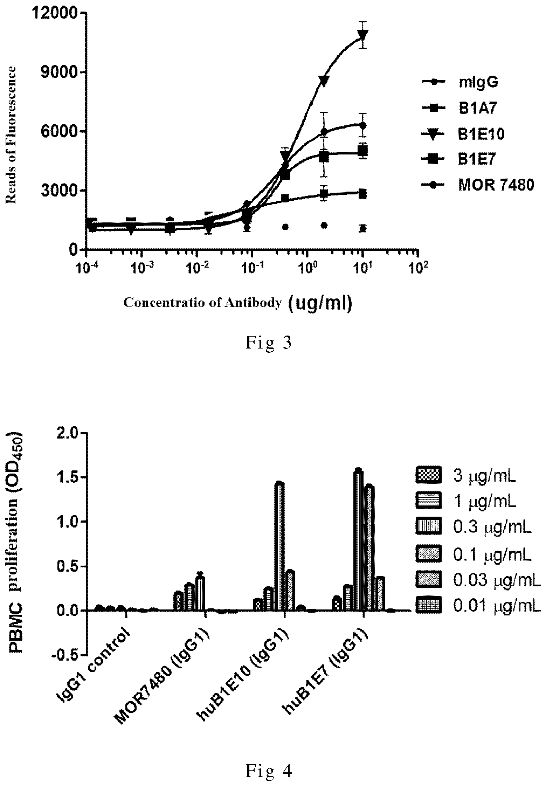

The stable cell strain HEK293F-h4-1BB-NFkB (Shanghai ChemPartner Chemistry) was thawed with DMEM/FBS medium, and the passaged cells were seeded into a 96-well plate at 4×10³ cells per well. Each antibody (including the test antibodies, the reference antibody, the IgG negative control, and so forth) was mixed with anti-F(ab')2 at a ratio, serially diluted, and incubated at 37 °C for 0.5 hours in a cell culture incubator. The antibodies were then added into the cell plate, incubated for 5 hours in the incubator, added with 100 µl of detection buffer to lyse the cells, and incubated at room temperature for 10 minutes in the dark. Fluorescence readings were measured with Envision and activity-concentration curves were plotted. The results of the three murine antibodies obtained from *in vitro* reporter assay were shown in Figure 3. The reporter reading value for B1E10 was stronger than that for PfizerreferenceMOR7480; whereas the value for B1E7 was slightly weaker than the reference.

### Example 5: The in vitro binding affinity and kinetics of the recombinant antibodies

Biacore is a well-known method for the objective detection of the affinity and kinetics of proteins. The present inventors analyzed the characteristic affinity and binding kinetics of the 4-1BB antibodies to be tested of this application via Biacore T200 (GE).

The recombinant anti-4-1BB antibodies to be tested of the present application were covalently linked toCM5 (GE) chip by NHS standard amino coupling method with a kit available from Biacore. Then, a series of gradient concentrations of human 4-1BB His protein (Sinobiological, Beijing #10041-H08H) diluted in the same buffer were injected at a flow rate of 10 µL/min. Regeneration was performed with regeneration reagents provided in the kit. Antigen-antibody binding kinetics were followed for 3 minutes and dissociation kinetics were followed for 10 minutes. The resulting data were analyzed using1:1 (Langmuir) binding model with BIAevaluation software available from GE. The ka (kₒₙ), kd (k_{off}) and K_{D} values of the chimeric antibodies determined by this method were shown in the table below.

**Table 7. Kinetic parameters of the antibodies**

| **Antibodies to be tested** | **ka (1/Ms)** | **kd (1/s)** | **K_{D} (M)** |
|---|---|---|---|
| ChB1E7 | 4.053E+6 | 5.997E-3 | 1.480E-9 |
| ChB1E10 | 1.282E+6 | 5.421E-4 | 4.228E-10 |
| MOR7480 (Pfizer) | 8.051E+5 | 3.359E-4 | 4.172E-10 |

### Example 6: Humanization of the mouse antibodies

In the present example, two strains (B1E10 and B1E7) having the strongest functional activity among the resulting murine antibodies were humanized. Based on the typical structure of the murine antibody V_{H}/V_{L}CDR obtained, the heavy and light chain variable region sequences were aligned against those disclosed in the antibody Germline database to obtain a human germline template with high homology.

The human germline light chain framework region was derived from human κ light chain gene, and the human germline light chain template used in the present application were preferably IGKV3-11*01 (for B1E10) and IGKV4-1*01 (for B1E7). The human germline heavy chain framework region was derived from human heavy chain, and the human germline heavy chain templates used in the present application were preferably IGHV3-30*01 (for B1E10) and IGHV1-46*01 (for B1E7).
Human germline heavy chain template IGHV3-30*01 (SEQ ID NO: 21): 
QVQLVESGGGVVQPGRSLRLSCAASGFTFSSYAMHWVRQAPGKGLEWVAVISY
DGSNKYYADSVKGRFTISRDNSKNTLYLQMNSLRAEDTAVYYCAR
Human germline light chain template IGKV3-11*01 (SEQ ID NO: 22): 
EIVLTQSPATLSLSPGERATLSCRASQSVSSYLAWYQQKPGQAPRLLIYDASNRAT
GIPARFSGSGSGTDFTLTISSLEPEDFAVYYCQQRSNWP
Human germline heavy chain template IGHV1-46*01 (SEQ ID NO: 23): 
QVQLVQSGAEVKKPGASVKVSCKASGYTFTSYYMHWVRQAPGQGLEWMGIIN
PSGGSTSYAQKFQGRVTMTRDTSTSTVYMELSSLRSEDTAVYYCAR
Human germline light chain template IGKV4-1*01 (SEQ ID NO: 24):

DIVMTQSPDSLAVSLGERATINCKSSQSVLYSSNNKNYLAWYQQKPGQPPKLLIY
WASTRESGVPDRFSGSGSGTDFTLTISSLQAEDVAVYYCQQYYSTP

The CDR regions of the murine antibody were grafted onto the selected humanized template, as a substitute for the humanized variable regions, and then recombined with IgG constant region. Then, based on the three-dimensional structure of the murine antibody, the embedding residues, the residues that directly interact with the CDR regions, and the residues that have an important influence on the conformation of VL and VH were subjected to back mutation. Further, for antibody B1E10, the chemically unstable amino acid residue in the CDR regions was optimized, i.e., HCDR2: TISDGGSYTYYPDNVKG (SEQ ID NO: 4) was optimized as: TIS**E**GGSYTYYPDNVKG (SEQ ID NO: 45).A series of humanized molecules were obtained.

The heavy chain variable region sequences of the two antibodies are shown in SEQ ID NOs: 25-27 and SEQ ID NOs: 31-33, respectively; and the light chain variable region sequences are set forth in SEQ ID NOs: 28-30 and SEQ ID NO: 34-36, respectively.
huB1E10-VH-a (SEQ ID NO: 25): 
EVQLVESGGGVVQPGRSLRLSCAASGFTFGDYYMYWVRQAPGKGLEWVATISE
GGSYTYYPDNVKGRFTISRDNSKNTLYLQMNSLRAEDTAVYYCARYYSKALYA
MDYWGRGTLVTVSS
huB1E10-VH-b (SEQ ID NO: 26): 
EVQLVESGGGVVQPGRSLRLSCAASGFTFGDYYMYWVRQAPGKGLEWVATISE
GGSYTYYPDNVKGRFTISRDNSKNTLYLQMNSLRAEDTAVYYCARYYSKALYA
MDYWGQGTLVTVSS
huB1E10-VH-c (SEQ ID NO: 27): 
EVQLVESGGGVVQPGRSLRLSCAASGFTFGDYYMYWVRQAPGKGLEWVATISE
GGSYTYYPDNVKGRFTISRDNAKNTLYLQMNSLRAEDTAVYYCARYYSKALYA
MDYWGRGTLVTVSS
huB1E10-VL-a (SEQ ID NO: 28): 
EIVLTQSPATLSLSPGERATLSCRASKSVSTSGFSYIHWYQQKPGQAPRLLIYTAS
NLESGIPARFSGSGSGTDFTLTISSLEPEDFAVYYCQHSRELPLTFGQGTKLEVK
huB1E10-VL-b (SEQ ID NO: 29): 
EIVLTQSPATLSLSPGERATLSCRASKSVSTSGFSYIHWYQQKPGQPPRLLIYTASN
LESGIPARFSGSGSGTDFTLTISSLEPEDFAVYYCQHSRELPLTFGQGTKLEVK
huB1E10-VL-c (SEQ ID NO: 30): 
EIVLTQSPATLSLSPGERATLSCRASKSVSTSGFSYIHWYQQKPGQPPRLLIYTASN
LESGVPARFSGSGSGTDFTLTISSLEPEDFAVYYCQHSRELPLTFGQGTKLEVK
huB1E7-VH-a (SEQ ID NO: 31): 
EVQLVQSGAEVKKPGSSVKVSCKASGYTFTSYGLNWVRQAPGQGLEWIGYINP
GSGYTKYNEKFEGRTTLTVDKSTSTVYMELSSLRSEDTAVYFCARWGLGRNWN
FAVWGTGTLVTVSS
huB1E7-VH-b (SEQ ID NO: 32): 
EVQLVQSGAEVKKPGSSVKVSCKASGYTFTSYGLNWVRQAPGQGLEWMGYIN
PGSGYTKYNEKFEGRTTLTVDKSTSTVYMELSSLRSEDTAVYFCARWGLGRNW
NFAVWGTGTLVTVSS
huB1E7-VH-c (SEQ ID NO: 33): 
EVQLVQSGAEVKKPGSSVKVSCKASGYTFTSYGLNWVRQAPGQGLEWMGYIN
PGSGYTKYNEKFEGRVTITADKSTSTAYMELSSLRSEDTAVYYCARWGLGRNW
NFAVWGQGTLVTVSS
huB1E7-VL-a (SEQ ID NO: 34): 
DIVLTQSPDSLAVSLGERATINCRASESVDSYGNTFMHWYQQKPGQPPKLLIYRA
SNLESGIPDRFSGSGSGTDFTLTISSLQAEDVAVYYCQQSNEDPLTFGQGTKLEIK
huB1E7-VL-b (SEQ ID NO: 35): DIVLTQSPDSLAVSLGERATINCRASESVDSYGNTFMHWYQQKPGQPPKLLIYRA SNLESGVPDRFSGSGSGTDFTLTISSLQAEDVAVYYCQQSNEDPLTFGQGTKLEIK
huB1E7-VL-c (SEQ ID NO: 36):

DIVMTQSPDSLAVSLGERATINCRASESVDSYGNTFMHWYQQKPGQPPKLLIYR
ASNLESGVPDRFSGSGSGTDFTLTISSLQAEDVAVYYCQQSNEDPLTFGQGTKLEI
K

After a small-scale expression test for the above combinations of each light and heavy chain (as described in Example 3), a comparison of the number of the back mutations, and a comprehensive evaluation, humanized huB1E10 (with VH-c heavy chain and VL-c light chain) and huB1E7 antibody molecule (with VH-c heavy chain and VL-c light chain) were finally selected. The intact light and heavy chain sequences (IgG1 subtype) of these antibodies are set forth in SEQ ID NOs: 17-20.
huB1E10 HC 
EVQLVESGGGVVQPGRSLRLSCAASGFTFGDYYMYWVRQAPGKGLEWVATISE
GGSYTYYPDNVKGRFTISRDNAKNTLYLQMNSLRAEDTAVYYCARYYSKALYA
MDYWGRGTLVTVSSASTKGPSVFPLAPCSRSTSESTAALGCLVKDYFPEPVTVS
WNSGALTSGVHTFPAVLQSSGLYSLSSVVTVPSSNFGTQTYTCNVDHKPSNTKV
DKTVERKCCVECPPCPAPPVAGPSVFLFPPKPKDTLMISRTPEVTCVVVDVSHED
PEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIA
VEWESNGQPENNYKTTPPMLDSDGSFFLYSKLTVDKSRWQQGNVFSCSVMHEA
LHNHYTQKSLSLSPGK
SEQID NO: 17
huB1E10 LC 
EIVLTQSPATLSLSPGERATLSCRASKSVSTSGFSYIHWYQQKPGQPPRLLIYTASN
LESGVPARFSGSGSGTDFTLTISSLEPEDFAVYYCQHSRELPLTFGQGTKLEVKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQID NO: 18
huB1E7 HC 
EVQLVQSGAEVKKPGSSVKVSCKASGYTFTSYGLNWVRQAPGQGLEWMGYIN
PGSGYTKYNEKFEGRVTITADKSTSTAYMELSSLRSEDTAVYYCARWGLGRNW
NFAVWGQGTLVTVSSASTKGPSVFPLAPCSRSTSESTAALGCLVKDYFPEPVTVS
WNSGALTSGVHTFPAVLQSSGLYSLSSVVTVPSSNFGTQTYTCNVDHKPSNTKV
DKTVERKCCVECPPCPAPPVAGPSVFLFPPKPKDTLMISRTPEVTCVVVDVSHED
PEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIA
VEWESNGQPENNYKTTPPMLDSDGSFFLYSKLTVDKSRWQQGNVFSCSVMHEA
LHNHYTQKSLSLSPGK
SEQID NO: 19
huB1E7 LC

DIVMTQSPDSLAVSLGERATINCRASESVDSYGNTFMHWYQQKPGQPPKLLIYR

ASNLESGVPDRFSGSGSGTDFTLTISSLQAEDVAVYYCQQSNEDPLTFGQGTKLEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQID NO: 20

### Example 7: Functional assay of the activation of PBMC cells in vitro

The cell viability in the presence of anti-4-1BB antibodies were detected via proliferation assay of the fresh human peripheral blood mononuclear cells (PBMCs).

Fresh human PBMCs were isolated from fresh blood containing anticoagulant heparin by Ficoll density gradient centrifugation. Various concentrations of anti-4-1BB antibody by gradient dilution and 3 µg/mL anti-CD3 antibody were coated in ELISA plate and incubated at 4 °C overnight. The plate was washed on the next day, added with peripheral blood mononuclear cells at a density of 2 × 10⁵/well, and incubated in an incubator at 37 °C, 5% CO₂ for 7 days. After the incubation, the proliferation of PBMCs was detected with a CCK-8 kit. The values of OD₄₅₀ were detected, and the EC₅₀ values were calculated from the OD₄₅₀ values.

As a result, as shown in Fig. 4, the humanized antibody huB1E7 and huB1E10 (both are IgG1 subtype) showed higher efficiency on activating PBMC proliferation, compared to the reference antibody MOR7480, wherein huB1E7 showed a stronger activation function.

### Example 8: Activity data of the humanized antibodies

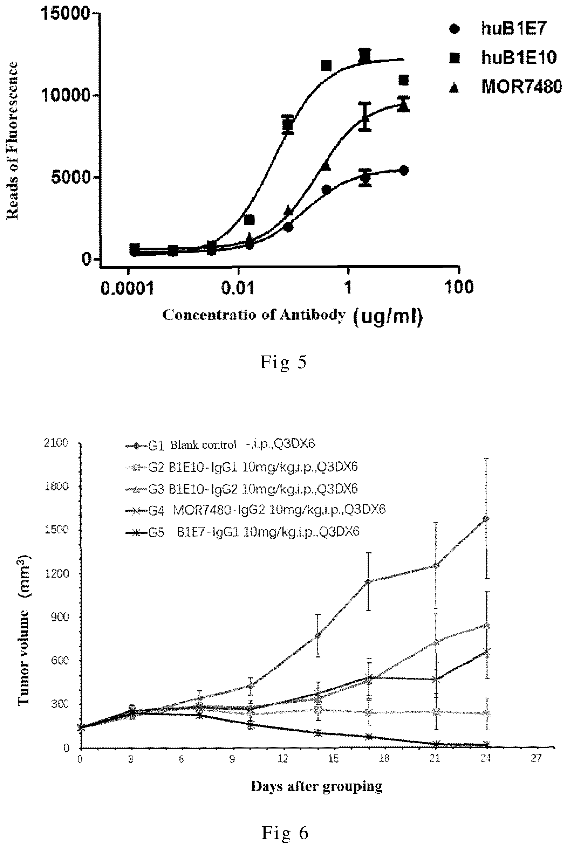

An *in vitro* reporter activity assay was performed for the humanized antibodies (the procedures were as described in Example 4). The results were shown in Figure 5. Consistent with the tendency as presented in murine antibodies, huB1E10 showed an increased activity of activating the reporter, when compared to the reference antibody MOR7480, while huB1E7 showed a weaker activity than the reference. This tendency was inconsistent with what was observed for the PBMC proliferation activity.

An *in vitro* affinity kinetic assay was performed for the humanized antibodies (the procedures were as described in Example 5) and the results were shown in the table below:

**Table 8. Affinity kinetics assay**

| **Antibodies to be tested** | **ka (1/Ms)** | **kd (1/s)** | **K_{D} (M)** |
|---|---|---|---|
| huB1E7 | 3.392E+6 | 6.627E-3 | 1.954E-9 |
| huB1E10 | 9.847E+6 | 2.630E-3 | 2.671E-10 |
| MOR7480 (Pfizer) | 8.051E+5 | 3.359E-4 | 4.172E-10 |

The results showed that both humanized antibody huB1E7 and huB1E10 retained binding activity to 4-1BB, wherein the affinity of huB1E10 was nearly twice than that of the reference antibody MOR7480, and the affinity of huB1E7 was slightly weaker.

### Example 9: Inhibition of growth of tumor cells in transgenic mice by theanti-4-1BB antibodies

The hu4-1BB transgenic C57BL/6 mice (female) were housed in an SPF facility environment at the Animal Center of Beijing Biotech Co., Ltd., using an independent ventilation cage with IVC. Mice were subjected to 3-7 days of adaptive feeding before the experiment. Temperature: 20-26 °C; Humidity: 40-70%; Lighting:12/12-hour light/dark cycle.

MC38 cells were purchased from Shanghai Shunran Biotechnology Co., Ltd., and the cells were cultured in Dulbecco's Modified Eagle Medium supplemented with 10% inactivated fetal bovine serum (Dulbecco's Modified Eagle' s Medium) in an incubator at 37 °C, 5% CO₂.

Cells were sub-cultured every 3 to 4 days, when approaching complete confluence. The hu4-1BB humanized homozygous mice were inoculated subcutaneously on the right flank with a concentration of 5 × 10⁶/ml MC38 tumor cells resuspended in PBS, 0.1 ml/mice, and a total of 50 mice were inoculated.

The tumor volumes were measured twice using a caliper, and the long and short diameters of the tumors were measured. The tumor volume was calculated as follows: the tumor volume (mm³) = 0.5 × (long diameter xshort diameter²).

Relative tumor inhibition rate TGI (%): TGI% = (1-T/C) × 100%. T/C% means the relative tumor increase rate, that is, it means the percentage of tumor volume or tumor weight of the treatment group relative to that of the control group atcertain time point. T and C are the tumor volume (TV) or tumor weight (TW) of the treatment group and that of IgG1 control group at a specific time point, respectively.

When the average tumor volume reached 140 mm³, the mice with moderate tumor volume were selected and randomly assigned to each experimental group according to the tumor volume. Drug administration was started on the day of grouping. The specific dosage regimen was shown in the following table. After the last administration, the body weight and tumor growth of the animals were observed for 9 days, then the experiment was ended. During the observation, tumor volume and animal body weight were measured twice a week, and the measured values were recorded, as shown in Table 10. At the end of the experiment, the tumor was removed from the mice and weighed. The animals were euthanized and the tumors were photographed.

**Table 9. Dosage regimen**

| **Group** | **Test drug** | **Number of animals (mice)** | **dosage (mg/kg)^{a}** | **Administration route** | **Administration frequency** | **Number of doses** |
|---|---|---|---|---|---|---|
| 1 | (PBS) control | 6 | -- | i.p. injection | Q3d | 6 |
| 2 | huB1E10-IgG1 | 6 | 10 | i.p. injection | Q3d | 6 |
| 3 | huB1E10-IgG2 | 6 | 10 | i.p. injection | Q3d | 6 |
| 4 | MOR7480-IgG2 | 6 | 10 | i.p. injection | Q3d | 6 |
| 5 | huB1E7-IgG1 | 6 | 10 | i.p. injection | Q3d | 6 |

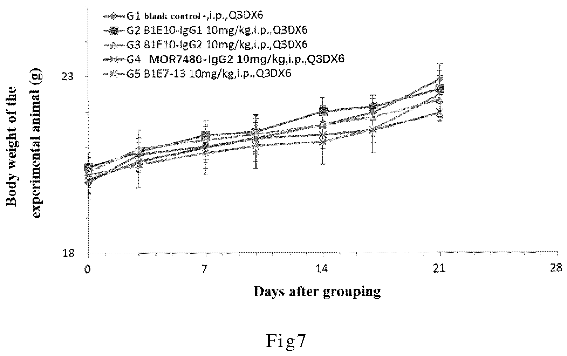

The results of the pharmacologic test were shown in Table 10, Figure 6 and Figure 7. The efficacy of antibody huB1E10-IgG2 subtype was close to that of Pfizer reference agentMOR7480 at the same dosage, while the efficacy of huB1E10-IgG1 subtype was significantly enhanced, while without significant effect on the body weight of mice.

The antibody huB1E7-IgG1 showed significantly more potent efficacy, wherein 2 out of 6 tumor-bearing mice showed complete elimination of tumors.

**Table 10. Tumor volume in mouse (mm³)**

| group | Animal No. | Days after inoculation | | | | | | | |
|---|---|---|---|---|---|---|---|---|---|
| | | 8 | 11 | 15 | 18 | 22 | 25 | 29 | 32 |
| | | Days after grouping | | | | | | | |
| | | 0 | 3 | 7 | 10 | 14 | 17 | 21 | 24 |
| G1 Blank control -,i.p.,Q3DX6 | 32328 | 125.9 | 205.7 | 367.4 | 483.4 | 877.3 | 1355.7 | 1141.7 | 1458.9 |
| | 32329 | 128.0 | 259.4 | 339.2 | 561.4 | 833.4 | 1157.0 | 1146.3 | 1179.1 |
| | 32333 | 120.0 | 127.2 | 217.0 | 225.8 | 327.8 | 513.8 | 527.1 | 765.5 |
| | 32338 | 124.5 | 207.5 | 551.6 | 554.0 | 1262.2 | 1736.6 | 1765.3 | 1625.0 |
| | 32355 | 157.1 | 343.0 | 388.3 | 424.3 | 944.0 | 1469.5 | 2390.5 | 3526.2 |
| | 32375 | 186.7 | 205.0 | 182.3 | 272.1 | 367.0 | 606.0 | 517.8 | 866.5 |
| | **mean** | 140 | 225 | 341 | 420 | 769 | 1140 | 1248 | 1570 |
| | **SD** | 26 | 72 | 133 | 143 | 359 | 488 | 728 | 1014 |
| | **SEM** | 11 | 29 | 54 | 58 | 147 | 199 | 297 | 414 |
| | **CV value** | 18.7% | 31.9% | 38.9% | 33.9% | 46.8% | 42.8% | 58.3% | 64.6% |
| G2 B1E10-IgG1 10mg/kg,i.p.,Q3D X6 | 32331 | 188.4 | 257.3 | 263.7 | 187.6 | 166.7 | 156.4 | 96.0 | 74.8 |
| | 32332 | 159.2 | 268.8 | 193.4 | 189.9 | 143.0 | 93.8 | 35.0 | 34.5 |
| | 32349 | 144.4 | 337.0 | 253.4 | 158.2 | 188.2 | 122.8 | 98.9 | 144.7 |
| | 32354 | 109.1 | 152.1 | 144.5 | 122.4 | 91.1 | 51.2 | 38.4 | 33.0 |
| | 32358 | 105.4 | 264.0 | 252.9 | 274.7 | 416.6 | 381.7 | 378.4 | 337.0 |
| | 32359 | 134.6 | 294.2 | 485.0 | 429.0 | 546.1 | 610.0 | 816.9 | 729.6 |
| | **mean** | 140 | 262 | 265 | 227 | 259 | 236 | 244 | 226 |
| | **SD** | 31 | 61 | 117 | 111 | 180 | 217 | 308 | 272 |
| | **SEM** | 13 | 25 | 48 | 45 | 74 | 88 | 126 | 111 |
| | **CV value** | 22.4% | 23.4% | 44.0% | 48.9% | 69.7% | 91.8% | 126.4% | 120.5% |
| | **TGI** | - | -0.449 | 0.375 | 0.690 | 0.811 | 0.904 | 0.906 | 0.940 |
| | **P value** | 0.991 | 0.352 | 0.320 | 0.026 | 0.011 | 0.002 | 0.011 | 0.011 |
| G3 B1E10-IgG2 10mg/kg,i.p.,Q3D X6 | 32340 | 140.2 | 226.4 | 296.5 | 283.2 | 292.0 | 612.3 | 934.0 | 1246.3 |
| | 32344 | 134.9 | 210.1 | 174.2 | 185.1 | 146.7 | 110.1 | 76.8 | 58.9 |
| | 32347 | 188.1 | 160.4 | 186.3 | 126.8 | 149.1 | 158.8 | 313.9 | 239.7 |
| | 32348 | 150.5 | 170.4 | 219.5 | 270.8 | 329.0 | 308.3 | 616.8 | 961.2 |
| | 32350 | 119.1 | 254.0 | 371.5 | 370.8 | 523.4 | 835.1 | 1272.3 | 1370.7 |
| | 32362 | 109.4 | 292.3 | 481.7 | 424.1 | 571.6 | 712.1 | 1130.9 | 1170.3 |
| | **mean** | 140 | 219 | 288 | 277 | 335 | 456 | 724 | 841 |
| | **SD** | 28 | 50 | 120 | 111 | 181 | 304 | 471 | 555 |
| | **SEM** | 11 | 20 | 49 | 45 | 74 | 124 | 192 | 227 |
| | **CV value** | 19.7% | 22.9% | 41.8% | 40.1% | 53.9% | 66.8% | 65.0% | 66.0% |
| | **TGI** | - | 0.068 | 0.263 | 0.512 | 0.690 | 0.684 | 0.473 | 0.510 |
| | **P value** | 1.000 | 0.876 | 0.488 | 0.080 | 0.025 | 0.015 | 0.169 | 0.153 |
| G4 MOR7480-IgG2 10mg/kg,i.p.,Q3D X6 | 32326 | 160.2 | 176.4 | 201.1 | 150.6 | 97.5 | 30.8 | 21.3 | 0.0 |
| | 32341 | 114.4 | 246.8 | 249.0 | 244.9 | 583.4 | 903.8 | 867.7 | 1211.2 |
| | 32357 | 188.2 | 440.1 | 520.3 | 453.1 | 452.2 | 548.0 | 586.7 | 760.4 |
| | 32360 | 126.5 | 216.9 | 236.9 | 276.4 | 554.6 | 714.2 | 647.2 | 1104.9 |
| | 32361 | 139.0 | 258.8 | 238.8 | 254.1 | 289.8 | 283.8 | 344.1 | 485.1 |
| | 32374 | 112.4 | 204.5 | 243.8 | 188.0 | 254.7 | 405.3 | 311.9 | 375.6 |
| | **mean** | 140 | 257 | 282 | 261 | 372 | 481 | 463 | 656 |
| | **SD** | 29 | 94 | 118 | 105 | 190 | 311 | 298 | 460 |
| | **SEM** | 12 | 39 | 48 | 43 | 78 | 127 | 122 | 188 |
| | **CV value** | 21.0% | 36.7% | 41.9% | 40.1% | 51.0% | 64.8% | 64.4% | 70.1% |
| | **TGI** | - | -0.390 | 0.294 | 0.567 | 0.631 | 0.659 | 0.708 | 0.639 |
| | **P value** | 0.987 | 0.516 | 0.432 | 0.052 | 0.038 | 0.019 | 0.035 | 0.072 |
| G5 B1E7-IgG1 10mg/kg,i.p.,Q3D X6 | 32345 | 169.6 | 236.5 | 153.4 | 98.6 | 72.6 | 42.5 | 25.7 | 22.8 |
| | 32351 | 140.5 | 161.6 | 207.2 | 95.3 | 96.3 | 84.3 | 25.7 | 0.0 |
| | 32352 | 172.9 | 376.7 | 289.9 | 272.4 | 161.7 | 123.9 | 54.8 | 51.1 |
| | 32365 | 130.8 | 188.3 | 210.4 | 137.8 | 58.6 | 42.7 | 0.0 | 0.0 |
| | 32368 | 124.7 | 214.9 | 231.2 | 120.2 | 66.2 | 52.4 | 0.0 | 0.0 |
| | 32371 | 102.8 | 230.3 | 252.8 | 203.1 | 140.0 | 76.3 | 14.7 | 0.0 |
| | **mean** | 140 | 235 | 224 | 155 | 99 | 70 | 20 | 12 |
| | **SD** | 27 | 75 | 46 | 70 | 42 | 31 | 20 | 21 |
| | **SEM** | 11 | 31 | 19 | 28 | 17 | 13 | 8 | 9 |
| | **CV value** | 19.3% | 31.9% | 20.6% | 45.2% | 42.8% | 44.7% | 101.8% | 171.1% |
| | **TGI** | - | -0.122 | 0.582 | 0.949 | 1.065 | 1.070 | 1.108 | 1.089 |
| | **P value** | 0.992 | 0.816 | 0.069 | 0.002 | 0.001 | 0.000 | 0.002 | 0.004 |

## Claims

1. An anti-4-1BB antibody or antigen-binding fragment thereof, wherein:
the antibody heavy chain variable region comprising
(I) HCDR1, HCDR2 and HCDR3 as set forth in SEQ ID NO: 3, SEQ ID NO: 4 and SEQ ID NO: 5, respectively; or
(II) HCDR1, HCDR2 and HCDR3 as set forth in SEQ ID NO: 3, SEQ ID NO: 45 and SEQ ID NO: 5, respectively; or
(III) HCDR1, HCDR2 and HCDR3 as set forth in SEQ ID NO: 11, SEQ ID NO: 12 and SEQ ID NO: 13, respectively; or
(IV) HCDR1, HCDR2 and HCDR3 as set forth in SEQ ID NO: 39, SEQ ID NO: 40 and SEQ ID NO: 41, respectively; and/or
the antibody light chain variable region comprising:
(I) LCDR1, LCDR2and LCDR3 as set forth in SEQ ID NO: 6, SEQ ID NO: 7and SEQ ID NO: 8, respectively; or
(II) LCDR1, LCDR2and LCDR3 as set forth in SEQ ID NO: 14, SEQ ID NO: 15and SEQ ID NO: 16, respectively; or
(III) LCDR1, LCDR2and LCDR3 as set forth in SEQ ID NO: 42, SEQ ID NO: 43and SEQ ID NO: 44, respectively.

2. The anti-4-1BB antibody or antigen-binding fragment thereof according to claim 1, which comprises any one selected from the group consisting of (I) to (IV):
(I) a heavy chain variable region comprising HCDR1, HCDR2 and HCDR3 as set forth in SEQ ID NO: 3, SEQ ID NO: 4 and SEQ ID NO: 5, respectively; and
a light chain variable region comprising LCDR1, LCDR2 and LCDR3 as set forth in SEQ ID NO: 6, SEQ ID NO: 7, and SEQ ID NO: 8, respectively;
(II) a heavy chain variable region comprising HCDR1, HCDR2 and HCDR3 as set forth in SEQ ID NO: 3, SEQ ID NO: 45 and SEQ ID NO: 5; and
a light chain variable region comprising LCDR1, LCDR2 and LCDR3 as set forth in SEQ ID NO: 6, SEQ ID NO: 7and SEQ ID NO: 8, respectively;
(III) a heavy chain variable region comprising HCDR1, HCDR2 and HCDR3 as set forth in SEQ ID NO: 11, SEQ ID NO: 12 and SEQ ID NO: 13, respectively; and
a light chain variable region comprising LCDR1, LCDR2 and LCDR3 as set forth in SEQ ID NO: 14, SEQ ID NO: 15and SEQ ID NO: 16, respectively;
(IV) a heavy chain variable region comprising HCDR1, HCDR2 and HCDR3 as set forth in SEQ ID NO: 39, SEQ ID NO: 40 and SEQ ID NO: 41, respectively; and
a light chain variable region comprising LCDR1, LCDR2, and LCDR3 as set forth in SEQ ID NO: 42, SEQ ID NO: 43and SEQ ID NO: 44, respectively.

3. The anti-4-1BB antibody or antigen-binding fragment according to any one of claims 1 to 2, wherein:
the heavy chain variable region is set forth in SEQ ID NO: 1 and the light chain variable region is set forth in SEQ ID NO: 2; or
the heavy chain variable region is set forth in SEQ ID NO: 9and the light chain variable region is set forth in SEQ ID NO: 10; or
the heavy chain variable region is set forth in SEQ ID NO: 37 and the light chain variable region is set forth in SEQ ID NO: 38.

4. The anti-4-1BB antibody or antigen-binding fragment thereof according to any one of claims 1 to 3, which is a murine antibody, a chimeric antibody, a human antibody, a humanized antibody or fragment thereof.

5. The anti-4-1BB antibody or antigen-binding fragment thereof according to claim 4, wherein the heavy chain framework region of the antibody or antigen-binding fragment thereof is derived from a heavy chain shown as the sequence selected from the group consisting of SEQ ID NO: 21 and SEQ ID NO: 23; and/or
the light chain framework region is derived from a light chain shown as the sequence selected from the group consisting of SEQ ID NO: 22 and SEQ ID NO: 24.

6. The anti-4-1BB antibody or antigen-binding fragment thereof according to any of the preceding claims, which comprises any one selected from the group consisting of (I) to (V):
(I) a heavy chain variable region, which is shown as the sequence selected from the group consisting of SEQ ID NOs: 25-27 and SEQ ID NOs: 31-33; and/or
a light chain variable region, which is shown as the sequence selected from the group consisting of SEQ ID NOs: 28-30 and SEQ ID NOs: 34-36;
(II) a heavy chain variable region, which is shown as the sequence selected from the group consisting of SEQ ID NOs: 25-27, and/or
a light chain variable region, which is shown as the sequence selected from the group consisting of SEQ ID NOs: 28-30;
(III) a heavy chain variable region, which is shown as the sequence selected from the group consisting of SEQ ID NOs: 31-33, and/or
a light chain variable region, which is shown as the sequence selected from the group consisting of SEQ ID NOs: 34-36;
(IV) a heavy chain variable region, which is set forth in SEQ ID NO: 27, and/or
a light chain variable region, which is set forth in SEQ ID NO:30; and
(V) a heavy chain variable region, which is set forth in SEQ ID NO: 33, and/or
a light chain variable region, which is set forth in SEQ ID NO:36.

7. The anti-4-1BB antibody or antigen-binding fragment thereof according to any of the preceding claims, which comprises any one selected from the group consisting of (I) to (V):
(I) a heavy chain of the antibody or antigen-binding fragment thereof, as set forth in SEQ ID NO: 17 or a variant sequence thereof, SEQ ID NO: 19 or a variant sequence thereof; and/or
a light chain as set forth in SEQ ID NO: 18 or a variant sequence thereof, SEQ ID NO: 20 or a variant sequence thereof;
(II) the antibody or antigen-binding fragment thereof according to (I), wherein the variant sequence comprises 0 to 10 amino acid changes in the light chain variable region, and/or comprises 0 to 10 amino acid changes in the heavy chain variable region;
(III) the antibody or antigen-binding fragment thereof according to (I) or (II), wherein:
the variant sequence of SEQ ID NO: 17 comprises any one of the mutations on positions 53, 75 and112 or any combination thereof; and/or
the variant sequence of SEQ ID NO: 18 comprises any one of the mutations on positions 47 and 62 or a combination thereof; and/or
the variant sequence of SEQ ID NO: 19 comprises any one of the mutations on positions 48, 95 and 112or any combination thereof; and/or
the variant sequence of SEQ ID NO: 20 comprises any one of the mutations on positions 4 and 62 or a combination thereof;
(IV) the antibody or antigen-binding fragment thereof according to (III), wherein
the variant sequence of SEQ ID NO: 17 comprises any one of the mutations E53D, A75S and R112Q or any combination thereof; and/or
the variant sequence of SEQ ID NO: 18 comprises any one of the mutations P47A and V62I or a combination thereof; and/or
the variant sequence of SEQ ID NO: 19 comprises any one of the mutations M48I, Y95F and Q112T or any combination thereof; and/or
the variant sequence of SEQ ID NO: 20 comprises any one of the mutations M4L and V62I or a combination thereof;
(V) the antibody or antigen-binding fragment thereof according to any one of (II) to (IV), wherein the mutation in SEQ ID NO: 17 is E53D.

8. The anti-4-1BB antibody or antigen-binding fragment thereof according to claim 4, wherein the heavy chain variable region of the humanized antibody comprises a heavy chain framework region of human IgG1, IgG2, IgG3 or IgG4 or a variant thereof; preferably, comprises a heavy chain framework region of human IgG1, IgG2 or IgG4; more preferably, comprises a heavy chain framework region of human IgGl or IgG2.

9. An isolated antibody or antigen-binding fragment thereof, which competes with the anti-4-1BB antibody or antigen-binding fragment thereof of any one of claims 1 to 8 for binding to 4-1BB or an epitope thereof.

10. A bispecific antibody or multispecific antibody, comprising a light chain variable region and a heavy chain variable region as defined in any one of claims 1-9.

11. A single chain antibody comprising a light chain variable region and a heavy chain variable region as defined in any one of claims 1-9.

12. An antibody-drug conjugate, wherein the antibody comprises a light chain variable region and a heavy chain variable region as defined in any one of claims 1-9.

13. A polynucleotide encoding the anti-4-1BB antibody or antigen-binding fragment thereof according to any one of claims 1 to 8, the isolated antibody or antigen-binding fragment thereof according to claim 9, the bispecific antibody or multispecific antibody according to claim 10, the single chain antibody according to claim 11, the antibody as defined in the antibody-drug conjugate according to claim 12, preferably, the polynucleotide is DNA or RNA.

14. An expression vector comprising the polynucleotide of claim 13,
preferably, the expression vector is a viral vector,
more preferably, the expression vector is an oncolytic viral vector.

15. A host cell transformed with the expression vector of claim 14,
preferably, the host cell is a bacterial, yeast, mammalian cell,
more preferably, the host cell is *Escherichiacoli, Pichiapastoris,* Chinese hamster ovary cell or human embryonic kidney 293 cell.

16. A method for preparing an anti-4-1BB antibody or antigen-binding fragment thereof, comprising the steps of:
expressing the anti-4-1BB antibody or antigen-binding fragment thereof in the host cell of claim 15; and
isolating the anti-4-1BB antibody or antigen-binding fragment thereof from the host cell.

17. A pharmaceutical composition comprising:
any one or any combination selected from the group consisting of the antibody or antigen-binding fragment thereof according to any one of claims 1 to 9, the bispecific or multispecific antibody of claim 10, the single chain antibody of claim 11, the antibody-drug conjugate of claim 12, the polynucleotide of claim 13 and the expression vector of claim 14; and
a pharmaceutically acceptable excipient, diluent or carrier.

18. Use of any one of the following in the preparation of a medicament or a pharmaceutical composition:
the antibody or antigen-binding fragment thereof according to any one of claims 1 to 9, the bispecific or multispecific antibody of claim 10, the single chain antibody of claim 11, the antibody-drug conjugate of claim 12, the polynucleotide of claim 13 and
the expression vector of claim 14, or any combination thereof, wherein:
the medicament or the pharmaceutical composition is for treating or preventing a 4-1BB-mediated or 4-1BBL-mediated disease or condition;
preferably, the disease is cancer;
more preferably, the cancer is selected from the group consisting of melanoma, breast cancer, ovarian cancer, prostate cancer, pancreatic cancer, kidney cancer, lung cancer, liver cancer, stomach cancer, colorectal cancer, bladder cancer, head and neck cancer, thyroid cancer, esophageal cancer, cervical cancer, sarcoma, multiple myeloma, leukemia, lymphoma, gallbladder cancer and glioblastoma.

19. A method of treating or preventing a 4-1BB-mediated or 4-1BBL-mediated disease or condition, comprising:
administering to a subject a therapeutically or prophylactically effective amount of the antibody or antigen-binding fragment thereof according to any one of claims 1 to 9, the bispecific or multispecific antibody of claim 10, the single chain antibody of claim 11, the antibody-drug conjugate of claim 12, the polynucleotide of claim 13, the expression vector of claim 14, or the pharmaceutical composition of claim 17, or any combination thereof,
preferably, the disease is cancer;
more preferably, the cancer is selected from the group consisting of melanoma, breast cancer, ovarian cancer, prostate cancer, pancreatic cancer, kidney cancer, lung cancer, liver cancer, stomach cancer, colorectal cancer, bladder cancer, head and neck cancer, thyroid cancer, esophageal cancer, cervical cancer, sarcoma, multiple myeloma, leukemia, lymphoma, gallbladder cancer and glioblastoma.


Sequence listing

<110>  JIANGSU HENGRUI MEDICINE CO., LTD.

<120> ANTI-4-1BB ANTIBODY, ANTIGEN-BINDING FRAGMENT THEREOF AND MEDICAL USE
THEREOF

<160>  45

<170>  SIPOSequenceListing 1.0

<210>  1
<211>  120
<212>  PRT
<213>  Mus musculus

<400>  1
Glu Val Gln Leu Val Glu Ser Gly Gly Gly Leu Val Glu Pro Gly Gly
1               5                   10                  15
Ser Leu Lys Leu Ser Cys Ala Ala Ser Gly Phe Thr Phe Gly Asp Tyr
            20                  25                  30
Tyr Met Tyr Trp Val Arg Gln Thr Pro Glu Lys Arg Leu Glu Trp Val
        35                  40                  45
Ala Thr Ile Ser Asp Gly Gly Ser Tyr Thr Tyr Tyr Pro Asp Asn Val
    50                  55                  60
Lys Gly Arg Phe Thr Ile Ser Arg Asp Asn Ala Lys Asn Ser Leu Asp
65                  70                  75                  80
Leu Gln Met Ser His Leu Lys Ser Glu Asp Thr Ala Met Tyr Tyr Cys
                85                  90                  95
Ala Arg Tyr Tyr Ser Lys Ala Leu Tyr Ala Met Asp Tyr Trp Gly Arg
            100                 105                 110
Gly Thr Ser Val Thr Val Ser Ser
        115                 120

<210>  2
<211>  111
<212>  PRT
<213>  Mus musculus

<400>  2
Asp Ile Val Leu Thr Gln Ser Pro Ala Ser Leu Ala Val Ser Leu Gly
1               5                   10                  15
Gln Arg Ala Thr Leu Ser Cys Arg Ala Ser Lys Ser Val Ser Thr Ser
            20                  25                  30
Gly Phe Ser Tyr Ile His Trp Tyr Gln Gln Lys Pro Gly Gln Pro Pro
        35                  40                  45
Lys Leu Leu Ile Tyr Thr Ala Ser Asn Leu Glu Ser Gly Val Pro Ala
    50                  55                  60
Arg Phe Ser Gly Ser Gly Ser Gly Thr Asp Phe Thr Leu Asn Ile His
65                  70                  75                  80
Pro Val Glu Glu Glu Asp Ala Ala Thr Tyr Tyr Cys Gln His Ser Arg
                85                  90                  95
Glu Leu Pro Leu Thr Phe Gly Ala Gly Thr Lys Leu Glu Val Lys
            100                 105                 110

<210>  3
<211>  10
<212>  PRT
<213>  Mus musculus

<400>  3
Gly Phe Thr Phe Gly Asp Tyr Tyr Met Tyr
1               5                   10

<210> 4
<211> 17
<212> PRT
<213> Mus musculus

<400> 4
Thr Ile Ser Asp Gly Gly Ser Tyr Thr Tyr Tyr Pro Asp Asn Val Lys
1               5                   10                  15
Gly

<210> 5
<211> 11
<212> PRT
<213> Mus musculus

<400> 5
Tyr Tyr Ser Lys Ala Leu Tyr Ala Met Asp Tyr
1               5                   10

<210> 6
<211> 15
<212> PRT
<213> Mus musculus

<400> 6
Arg Ala Ser Lys Ser Val Ser Thr Ser Gly Phe Ser Tyr Ile His
1               5                   10                  15

<210> 7
<211> 7
<212> PRT
<213> Mus musculus

<400> 7
Thr Ala Ser Asn Leu Glu Ser
1               5

<210> 8
<211> 9
<212> PRT
<213> Mus musculus

<400> 8
Gln His Ser Arg Glu Leu Pro Leu Thr
1               5

<210> 9
<211> 120
<212> PRT
<213> Mus musculus

<400> 9
Glu Val Gln Leu Gln Gln Ser Gly Ala Glu Leu Val Arg Ala Gly Ser
1               5                   10                  15
Ser Val Glu Met Ser Cys Lys Ala Ser Gly Tyr Thr Phe Thr Ser Tyr
            20                  25                  30
Gly Leu Asn Trp Val Lys Gln Arg Pro Gly Gln Gly Leu Glu Trp Ile
        35                  40                  45
Gly Tyr Ile Asn Pro Gly Ser Gly Tyr Thr Lys Tyr Asn Glu Lys Phe
    50                  55                  60
Glu Gly Lys Thr Thr Leu Thr Val Asp Lys Ser Ser Ser Thr Val Tyr

65                  70                  75                  80
Met Gln Leu Arg Ser Leu Thr Ser Glu Asp Ser Ala Val Tyr Phe Cys
                85                  90                  95
Ala Arg Trp Gly Leu Gly Arg Asn Trp Asn Phe Ala Val Trp Gly Thr
            100                 105                 110
Gly Thr Thr Val Thr Val Ser Ser
        115                 120

<210>  10
<211>  111
<212>  PRT
<213>  Mus musculus

<400>  10
Asp Ile Val Leu Thr Gln Ser Pro Ala Ser Leu Ala Val Ser Leu Gly
1               5                   10                  15
Gln Arg Ala Thr Met Ser Cys Arg Ala Ser Glu Ser Val Asp Ser Tyr
            20                  25                  30
Gly Asn Thr Phe Met His Trp Tyr Gln Gln Lys Pro Gly Gln Pro Pro
        35                  40                  45
Lys Leu Leu Ile Tyr Arg Ala Ser Asn Leu Glu Ser Gly Ile Pro Ala
    50                  55                  60
Arg Phe Ser Gly Ser Gly Ser Gly Thr Asp Phe Thr Leu Thr Ile Asn
65                  70                  75                  80
Pro Val Glu Ala Asp Asp Val Ala Thr Tyr Tyr Cys Gln Gln Ser Asn
                85                  90                  95
Glu Asp Pro Leu Thr Phe Gly Ser Gly Thr Lys Leu Glu Ile Lys
            100                 105                 110

<210>  11
<211>  10
<212>  PRT
<213>  Mus musculus

<400>  11
Gly Tyr Thr Phe Thr Ser Tyr Gly Leu Asn
1               5                   10

<210>  12
<211>  17
<212>  PRT
<213>  Mus musculus

<400>  12
Tyr Ile Asn Pro Gly Ser Gly Tyr Thr Lys Tyr Asn Glu Lys Phe Glu
1               5                   10                  15
Gly


<210>  13
<211>  11
<212>  PRT
<213>  Mus musculus

<400>  13
Trp Gly Leu Gly Arg Asn Trp Asn Phe Ala Val
1               5                   10

<210>  14
<211>  15
<212>  PRT
<213>  Mus musculus

<400>  14
Arg Ala Ser Glu Ser Val Asp Ser Tyr Gly Asn Thr Phe Met His
1               5                   10                  15

<210>  15
<211>  7
<212>  PRT
<213>  Mus musculus

<400>  15
Arg Ala Ser Asn Leu Glu Ser
1               5

<210>  16
<211>  9
<212>  PRT
<213>  Mus musculus

<400>  16
Gln Gln Ser Asn Glu Asp Pro Leu Thr
1               5

<210>  17
<211>  446
<212>  PRT
<213>  Artificial Sequence

<220>
<223>  heavy chain

<400>  17
Glu Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Gly Asp Tyr
            20                  25                  30
Tyr Met Tyr Trp Val Arg Gln Ala Pro Gly Lys Gly Leu Glu Trp Val
        35                  40                  45
Ala Thr Ile Ser Glu Gly Gly Ser Tyr Thr Tyr Tyr Pro Asp Asn Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Tyr Tyr Ser Lys Ala Leu Tyr Ala Met Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Asn Phe Gly Thr Gln Thr Tyr Thr Cys Asn Val Asp His Lys
        195                 200                 205
Pro Ser Asn Thr Lys Val Asp Lys Thr Val Glu Arg Lys Cys Cys Val
    210                 215                 220
Glu Cys Pro Pro Cys Pro Ala Pro Pro Val Ala Gly Pro Ser Val Phe
225                 230                 235                 240
Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Phe Arg Val Val Ser Val
    290                 295                 300
Leu Thr Val Val His Gln Asp Trp Leu Asn Gly Lys Glu Tyr Lys Cys
305                 310                 315                 320
Lys Val Ser Asn Lys Gly Leu Pro Ala Pro Ile Glu Lys Thr Ile Ser
                325                 330                 335
Lys Thr Lys Gly Gln Pro Arg Glu Pro Gln Val Tyr Thr Leu Pro Pro
            340                 345                 350
Ser Arg Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Met Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210>  18
<211>  218
<212>  PRT
<213>  Artificial Sequence

<220>
<223>  light chain

<400>  18
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Lys Ser Val Ser Thr Ser
            20                  25                  30
Gly Phe Ser Tyr Ile His Trp Tyr Gln Gln Lys Pro Gly Gln Pro Pro
        35                  40                  45
Arg Leu Leu Ile Tyr Thr Ala Ser Asn Leu Glu Ser Gly Val Pro Ala
    50                  55                  60
Arg Phe Ser Gly Ser Gly Ser Gly Thr Asp Phe Thr Leu Thr Ile Ser
65                  70                  75                  80
Ser Leu Glu Pro Glu Asp Phe Ala Val Tyr Tyr Cys Gln His Ser Arg
                85                  90                  95
Glu Leu Pro Leu Thr Phe Gly Gln Gly Thr Lys Leu Glu Val Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  19
<211>  446
<212>  PRT
<213>  Artificial Sequence

<220>
<223>  heavy chain

<400>  19
Glu Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Ser Tyr
            20                  25                  30
Gly Leu Asn Trp Val Arg Gln Ala Pro Gly Gln Gly Leu Glu Trp Met
        35                  40                  45
Gly Tyr Ile Asn Pro Gly Ser Gly Tyr Thr Lys Tyr Asn Glu Lys Phe
    50                  55                  60
Glu Gly Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Leu Gly Arg Asn Trp Asn Phe Ala Val Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Asn Phe Gly Thr Gln Thr Tyr Thr Cys Asn Val Asp His Lys
        195                 200                 205
Pro Ser Asn Thr Lys Val Asp Lys Thr Val Glu Arg Lys Cys Cys Val
    210                 215                 220
Glu Cys Pro Pro Cys Pro Ala Pro Pro Val Ala Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Phe Arg Val Val Ser Val
    290                 295                 300
Leu Thr Val Val His Gln Asp Trp Leu Asn Gly Lys Glu Tyr Lys Cys
305                 310                 315                 320
Lys Val Ser Asn Lys Gly Leu Pro Ala Pro Ile Glu Lys Thr Ile Ser
                325                 330                 335
Lys Thr Lys Gly Gln Pro Arg Glu Pro Gln Val Tyr Thr Leu Pro Pro
            340                 345                 350
Ser Arg Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Met Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  20
<211>  218
<212>  PRT
<213>  Artificial Sequence

<220>
<223>  light chain

<400>  20
Asp Ile Val Met Thr Gln Ser Pro Asp Ser Leu Ala Val Ser Leu Gly
1               5                   10                  15
Glu Arg Ala Thr Ile Asn Cys Arg Ala Ser Glu Ser Val Asp Ser Tyr
            20                  25                  30
Gly Asn Thr Phe Met His Trp Tyr Gln Gln Lys Pro Gly Gln Pro Pro
        35                  40                  45
Lys Leu Leu Ile Tyr Arg Ala Ser Asn Leu Glu Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln Gln Ser Asn
                85                  90                  95
Glu Asp Pro Leu Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  21
<211>  98
<212>  PRT
<213>  Homo sapiens

<400>  21
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Ala Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg

<210>  22

<211>  95
<212>  PRT
<213>  Homo sapiens

<400>  22
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp Pro
                85                  90                  95

<210>  23
<211>  98
<212>  PRT
<213>  Homo sapiens

<400>  23
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Tyr Met His Trp Val Arg Gln Ala Pro Gly Gln Gly Leu Glu Trp Met
        35                  40                  45
Gly Ile Ile Asn Pro Ser Gly Gly Ser Thr Ser Tyr Ala Gln Lys Phe
    50                  55                  60
Gln Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg

<210>  24
<211>  101
<212>  PRT
<213>  Homo sapiens

<400>  24
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Ser
            20                  25                  30
Ser Asn Asn Lys Asn Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Ser Thr Pro
            100

<210>  25
<211>  120
<212>  PRT

<213>  Artificial Sequence

<220>
<223>  huB1E10-VH-a

<400>  25
Glu Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Gly Asp Tyr
            20                  25                  30
Tyr Met Tyr Trp Val Arg Gln Ala Pro Gly Lys Gly Leu Glu Trp Val
        35                  40                  45
Ala Thr Ile Ser Glu Gly Gly Ser Tyr Thr Tyr Tyr Pro Asp Asn Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Tyr Tyr Ser Lys Ala Leu Tyr Ala Met Asp Tyr Trp Gly Arg
            100                 105                 110
Gly Thr Leu Val Thr Val Ser Ser
        115                 120

<210>  26
<211>  120
<212>  PRT
<213>  Artificial Sequence

<220>
<223>  huB1E10-VH-b

<400>  26
Glu Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Gly Asp Tyr
            20                  25                  30
Tyr Met Tyr Trp Val Arg Gln Ala Pro Gly Lys Gly Leu Glu Trp Val
        35                  40                  45
Ala Thr Ile Ser Glu Gly Gly Ser Tyr Thr Tyr Tyr Pro Asp Asn Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Tyr Tyr Ser Lys Ala Leu Tyr Ala Met Asp Tyr Trp Gly Gln
            100                 105                 110
Gly Thr Leu Val Thr Val Ser Ser
        115                 120

<210>  27
<211>  120
<212>  PRT
<213>  Artificial Sequence

<220>
<223>  huB1E10-VH-c

<400>  27
Glu Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Gly Asp Tyr
            20                  25                  30
Tyr Met Tyr Trp Val Arg Gln Ala Pro Gly Lys Gly Leu Glu Trp Val

35                  40                  45
Ala Thr Ile Ser Glu Gly Gly Ser Tyr Thr Tyr Tyr Pro Asp Asn Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Tyr Tyr Ser Lys Ala Leu Tyr Ala Met Asp Tyr Trp Gly Arg
            100                 105                 110
Gly Thr Leu Val Thr Val Ser Ser
        115                 120

<210>  28
<211>  111
<212>  PRT
<213>  Artificial Sequence

<220>
<223>  huB1E10-VL-a

<400>  28
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Lys Ser Val Ser Thr Ser
            20                  25                  30
Gly Phe Ser Tyr Ile His Trp Tyr Gln Gln Lys Pro Gly Gln Ala Pro
        35                  40                  45
Arg Leu Leu Ile Tyr Thr Ala Ser Asn Leu Glu Ser Gly Ile Pro Ala
    50                  55                  60
Arg Phe Ser Gly Ser Gly Ser Gly Thr Asp Phe Thr Leu Thr Ile Ser
65                  70                  75                  80
Ser Leu Glu Pro Glu Asp Phe Ala Val Tyr Tyr Cys Gln His Ser Arg
                85                  90                  95
Glu Leu Pro Leu Thr Phe Gly Gln Gly Thr Lys Leu Glu Val Lys
            100                 105                 110

<210>  29
<211>  111
<212>  PRT
<213>  Artificial Sequence

<220>
<223>  huB1E10-VL-b

<400>  29
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Lys Ser Val Ser Thr Ser
            20                  25                  30
Gly Phe Ser Tyr Ile His Trp Tyr Gln Gln Lys Pro Gly Gln Pro Pro
        35                  40                  45
Arg Leu Leu Ile Tyr Thr Ala Ser Asn Leu Glu Ser Gly Ile Pro Ala
    50                  55                  60
Arg Phe Ser Gly Ser Gly Ser Gly Thr Asp Phe Thr Leu Thr Ile Ser
65                  70                  75                  80
Ser Leu Glu Pro Glu Asp Phe Ala Val Tyr Tyr Cys Gln His Ser Arg
                85                  90                  95
Glu Leu Pro Leu Thr Phe Gly Gln Gly Thr Lys Leu Glu Val Lys
            100                 105                 110

<210>  30
<211>  111
<212>  PRT

<213>  Artificial Sequence

<220>
<223>  huB1E10-VL-c

<400>  30
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Lys Ser Val Ser Thr Ser
            20                  25                  30
Gly Phe Ser Tyr Ile His Trp Tyr Gln Gln Lys Pro Gly Gln Pro Pro
        35                  40                  45
Arg Leu Leu Ile Tyr Thr Ala Ser Asn Leu Glu Ser Gly Val Pro Ala
    50                  55                  60
Arg Phe Ser Gly Ser Gly Ser Gly Thr Asp Phe Thr Leu Thr Ile Ser
65                  70                  75                  80
Ser Leu Glu Pro Glu Asp Phe Ala Val Tyr Tyr Cys Gln His Ser Arg
                85                  90                  95
Glu Leu Pro Leu Thr Phe Gly Gln Gly Thr Lys Leu Glu Val Lys
            100                 105                 110

<210>  31
<211>  120
<212>  PRT
<213>  Artificial Sequence

<220>
<223>  huB1E7-VH-a

<400>  31
Glu Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Ser Tyr
            20                  25                  30
Gly Leu Asn Trp Val Arg Gln Ala Pro Gly Gln Gly Leu Glu Trp Ile
        35                  40                  45
Gly Tyr Ile Asn Pro Gly Ser Gly Tyr Thr Lys Tyr Asn Glu Lys Phe
    50                  55                  60
Glu Gly Arg Thr Thr Leu Thr Val Asp Lys Ser Thr Ser Thr Val Tyr
65                  70                  75                  80
Met Glu Leu Ser Ser Leu Arg Ser Glu Asp Thr Ala Val Tyr Phe Cys
                85                  90                  95
Ala Arg Trp Gly Leu Gly Arg Asn Trp Asn Phe Ala Val Trp Gly Thr
            100                 105                 110
Gly Thr Leu Val Thr Val Ser Ser
        115                 120

<210>  32
<211>  120
<212>  PRT
<213>  Artificial Sequence

<220>
<223>  huB1E7-VH-b

<400>  32
Glu Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Ser Tyr
            20                  25                  30
Gly Leu Asn Trp Val Arg Gln Ala Pro Gly Gln Gly Leu Glu Trp Met
        35                  40                  45
Gly Tyr Ile Asn Pro Gly Ser Gly Tyr Thr Lys Tyr Asn Glu Lys Phe

50                  55                  60
Glu Gly Arg Thr Thr Leu Thr Val Asp Lys Ser Thr Ser Thr Val Tyr
65                  70                  75                  80
Met Glu Leu Ser Ser Leu Arg Ser Glu Asp Thr Ala Val Tyr Phe Cys
                85                  90                  95
Ala Arg Trp Gly Leu Gly Arg Asn Trp Asn Phe Ala Val Trp Gly Thr
            100                 105                 110
Gly Thr Leu Val Thr Val Ser Ser
        115                 120

<210>  33
<211>  120
<212>  PRT
<213>  Artificial Sequence

<220>
<223>  huB1E7-VH-c

<400>  33
Glu Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Ser Tyr
            20                  25                  30
Gly Leu Asn Trp Val Arg Gln Ala Pro Gly Gln Gly Leu Glu Trp Met
        35                  40                  45
Gly Tyr Ile Asn Pro Gly Ser Gly Tyr Thr Lys Tyr Asn Glu Lys Phe
    50                  55                  60
Glu Gly Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Leu Gly Arg Asn Trp Asn Phe Ala Val Trp Gly Gln
            100                 105                 110
Gly Thr Leu Val Thr Val Ser Ser
        115                 120

<210>  34
<211>  111
<212>  PRT
<213>  Artificial Sequence

<220>
<223>  huB1E7-VL-a

<400>  34
Asp Ile Val Leu Thr Gln Ser Pro Asp Ser Leu Ala Val Ser Leu Gly
1               5                   10                  15
Glu Arg Ala Thr Ile Asn Cys Arg Ala Ser Glu Ser Val Asp Ser Tyr
            20                  25                  30
Gly Asn Thr Phe Met His Trp Tyr Gln Gln Lys Pro Gly Gln Pro Pro
        35                  40                  45
Lys Leu Leu Ile Tyr Arg Ala Ser Asn Leu Glu Ser Gly Ile Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln Gln Ser Asn
                85                  90                  95
Glu Asp Pro Leu Thr Phe Gly Gln Gly Thr Lys Leu Glu Ile Lys
            100                 105                 110

<210>  35
<211>  111
<212>  PRT

<213>  Artificial Sequence

<220>
<223>  huB1E7-VL-b

<400>  35
Asp Ile Val Leu Thr Gln Ser Pro Asp Ser Leu Ala Val Ser Leu Gly
1               5                   10                  15
Glu Arg Ala Thr Ile Asn Cys Arg Ala Ser Glu Ser Val Asp Ser Tyr
            20                  25                  30
Gly Asn Thr Phe Met His Trp Tyr Gln Gln Lys Pro Gly Gln Pro Pro
        35                  40                  45
Lys Leu Leu Ile Tyr Arg Ala Ser Asn Leu Glu Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln Gln Ser Asn
                85                  90                  95
Glu Asp Pro Leu Thr Phe Gly Gln Gly Thr Lys Leu Glu Ile Lys
            100                 105                 110

<210>  36
<211>  111
<212>  PRT
<213>  Artificial Sequence

<220>
<223>  huB1E7-VL-c

<400>  36
Asp Ile Val Met Thr Gln Ser Pro Asp Ser Leu Ala Val Ser Leu Gly
1               5                   10                  15
Glu Arg Ala Thr Ile Asn Cys Arg Ala Ser Glu Ser Val Asp Ser Tyr
            20                  25                  30
Gly Asn Thr Phe Met His Trp Tyr Gln Gln Lys Pro Gly Gln Pro Pro
        35                  40                  45
Lys Leu Leu Ile Tyr Arg Ala Ser Asn Leu Glu Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln Gln Ser Asn
                85                  90                  95
Glu Asp Pro Leu Thr Phe Gly Gln Gly Thr Lys Leu Glu Ile Lys
            100                 105                 110

<210>  37
<211>  118
<212>  PRT
<213>  Mus musculus

<400>  37
Gln Val Thr Leu Lys Glu Ser Gly Pro Gly Ile Leu Gln Pro Ser Gln
1               5                   10                  15
Thr Leu Ser Leu Thr Cys Ser Phe Ser Gly Phe Ser Leu Ser Thr Ser
            20                  25                  30
Gly Met Gly Val Gly Trp Ile Arg Gln Pro Ser Gly Lys Gly Leu Glu
        35                  40                  45
Trp Leu Ala His Ile Trp Trp Asp Asp Val Lys Arg Tyr Asn Pro Ala
    50                  55                  60
Leu Lys Ser Arg Leu Thr Ile Ser Lys Asp Thr Ser Ser Ser Gln Val
65                  70                  75                  80
Phe Leu Lys Ile Ala Ser Val Asp Thr Ala Asp Thr Ala Thr Tyr Tyr
                85                  90                  95

Cys Ala Gln Met Thr Ser Ser Val Phe Ala Tyr Trp Gly Gln Gly Thr
            100                 105                 110
Leu Val Thr Val Ser Ala
        115

<210>  38
<211>  111
<212>  PRT
<213>  Mus musculus

<400>  38
Asp Ile Val Leu Thr Gln Ser Pro Asp Ser Leu Ala Val Ser Leu Gly
1               5                   10                  15
Gln Arg Ala Thr Ile Ser Cys Arg Ala Ser Glu Ser Val Asp Ser Tyr
            20                  25                  30
Gly His Ser Phe Met His Trp Tyr Gln Gln Lys Pro Gly Gln Pro Pro
        35                  40                  45
Lys Leu Leu Ile Tyr Arg Ala Ser Asn Leu Glu Ser Gly Ile Pro Ala
    50                  55                  60
Arg Phe Ser Gly Ser Gly Ser Arg Thr Asp Phe Thr Leu Thr Ile Asn
65                  70                  75                  80
Val Val Glu Ala Asp Asp Val Ala Thr Tyr Tyr Cys His Gln Ser Tyr
                85                  90                  95
Glu Glu Pro Trp Thr Phe Gly Gly Gly Thr Lys Leu Glu Ile Lys
            100                 105                 110

<210>  39
<211>  12
<212>  PRT
<213>  Mus musculus

<400>  39
Gly Phe Ser Leu Ser Thr Ser Gly Met Gly Val Gly
1               5                   10

<210>  40
<211>  16
<212>  PRT
<213>  Mus musculus

<400>  40
His Ile Trp Trp Asp Asp Val Lys Arg Tyr Asn Pro Ala Leu Lys Ser
1               5                   10                  15

<210>  41
<211>  8
<212>  PRT
<213>  Mus musculus

<400>  41
Met Thr Ser Ser Val Phe Ala Tyr
1               5

<210>  42
<211>  15
<212>  PRT
<213>  Mus musculus

<400>  42
Arg Ala Ser Glu Ser Val Asp Ser Tyr Gly His Ser Phe Met His
1               5                   10                  15

<210>  43

<211>  7
<212>  PRT
<213>  Mus musculus

<400>  43
Arg Ala Ser Asn Leu Glu Ser
1               5

<210>  44
<211>  9
<212>  PRT
<213>  Mus musculus

<400>  44
His Gln Ser Tyr Glu Glu Pro Trp Thr
1               5

<210>  45
<211>  17
<212>  PRT
<213>  Mus musculus

<400>  45
Thr Ile Ser Glu Gly Gly Ser Tyr Thr Tyr Tyr Pro Asp Asn Val Lys
1               5                   10                  15
Gly